(19) Europäisches Patentamt / European Patent Office / Office européen des brevets

(11) **EP 4 674 975 A1**

(12) **EUROPEAN PATENT APPLICATION**
published in accordance with Art. 153(4) EPC

(43) Date of publication:
07.01.2026 Bulletin 2026/02

(21) Application number: 24764019.6

(22) Date of filing: 29.02.2024

(51) International Patent Classification (IPC):
*C12Q 1/48* (2006.01)     *C12Q 1/02* (2006.01)
*G01N 33/15* (2006.01)     *G01N 33/50* (2006.01)
*G01N 33/573* (2006.01)

(52) Cooperative Patent Classification (CPC):
C12Q 1/02; C12Q 1/48; G01N 33/15; G01N 33/50;
G01N 33/573

(86) International application number:
PCT/JP2024/007599

(87) International publication number:
WO 2024/181542 (06.09.2024 Gazette 2024/36)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 01.03.2023 JP 2023031358

(71) Applicants:
• The University of Osaka
Osaka 565-0871 (JP)

• RIKEN
Wako-shi, Saitama 351-0198 (JP)

(72) Inventors:
• UEDA, Masahiro
Suita-shi, Osaka 565-0871 (JP)
• HIROSHIMA, Michio
Wako-shi, Saitama 351-0198 (JP)
• WATANABE, Daisuke
Suita-shi, Osaka 565-0871 (JP)

(74) Representative: Dehns
10 Old Bailey
London EC4M 7NG (GB)

(54) **METHOD FOR EVALUATING RECEPTOR-TYPE TYROSINE KINASE ACTIVITY**

(57) In one aspect, a novel method that allows for evaluation of an RTK activity is provided. Provided is a method for evaluating an RTK activity, the method including: a step (A1) for determining diffusion dynamics A1 of the RTK by contacting a target substance with a cell that expresses a labeled RTK on a cell membrane, a step (B1) for determining diffusion dynamics B1 of the RTK by contacting the target substance and a ligand that activates the RTK with the cell that expresses the labeled RTK on a cell membrane, a step (C1) for calculating a ratio of the diffusion dynamics A1 of the RTK obtained at the step (A1) and the diffusion dynamics B1 of the RTK obtained at the step (B1), and a step (D1) for evaluating an effect of the target substance on an RTK activity by comparing the ratio of the diffusion dynamics obtained at the step (C1) with a threshold; and at the step (D1), when the ratio of the diffusion dynamics obtained at the step (C1) is equal to or greater than the threshold, it can indicate that the target substance has an effect on a molecular function of the RTK.

FIG. 5

EP 4 674 975 A1

## Description

Field

The present disclosure relates to a method for evaluating an activity of a receptor tyrosine kinase (RTK) and a method for screening a candidate inhibitor or activator that targets the RTK.

Background

A receptor tyrosine kinase (RTK) is a receptor that functions on a cell membrane and transduces a signal from outside a cell into the cell through its own phosphorylation, diffusion movement, multimer formation, and intracellular uptake. One of the representative molecules of RTKs is an epidermal growth factor receptor (EGFR). When an EGFR binds to an EGF, which is an extracellular ligand, it undergoes autophosphorylation in the resulting dimer and then transduces an extracellular signal into the cell through complex reaction processes that include formation of a multimer. Subsequently, the EGFR stops signaling on the membrane by being incorporated into the cell.

Genetic mutation in the EGFR is found in various cancer cells and tissues, including lung cancer, and it is a major cause of cancer development; therefore, development of molecularly targeted drugs for the EGFR is underway. In the development of molecularly targeted drugs for the EGFR, compound/drug screening has been conventionally conducted using inhibition of the EGFR's phosphorylation activity (kinase activity) as an indicator.

By using cellular single-molecule fluorescence imaging to visualize a membrane protein such as a receptor on a living cell membrane, the inventors of the present invention have found a correspondence relationship between an intermolecular interaction, which commonly occurs in a G protein-coupled receptor (GPCR), and diffusion dynamics, and filed a patent application with regard to a method for evaluating GPCR's activity by measuring an average diffusion coefficient of the GPCR (Patent Literature 1).

The inventors of the present invention have also developed an automated microscopy system for cellular single-molecule fluorescence imaging, and have attempted single-molecule analysis by fluorescently labeling the EGFR, one of the representative RTK molecules, tracking changes in the EGFR diffusion movement and multimer formation during a series of signaling processes at a single-molecule level (Non-Patent Literature 1).

Citation List

Patent Literature

Patent Literature 1: Japanese Patent Application Laid-open No. 2018-179946

Non Patent Literature

Non-Patent Literature 1: Masato Yasui et al., nature commun (2018) doi: s41467-018-05524-7 (1)

Summary

Technical Problem

Conventionally, drug screening for the RTK including the EGFR has been conducted using inhibition of the phosphorylation activity (kinase activity) of the RTK including the EGFR as an indicator. However, the RTK including the EGFR transduces a signal not only through the phosphorylation but also through a diffusion across a cell membrane, a multimer formation, and an intracellular uptake. Therefore, although conventional methods can screen the drug that inhibits the kinase activity, they are not sufficient to screen the drug that affects other signaling processes. Accordingly, it has been desired to develop a screening method that can evaluate not only the EGFR kinase activity on a cell membrane but also other signaling processes of the RTK represented by the EGFR.

During the course of attempting to track the change in the diffusion dynamics and multimer formation of the EGFR at a single-molecule level, the inventors of the present invention have found that the mean square displacement (movement) of the EGFR changes as the concentration of a ligand EGF and other drugs (target substances) increases, and that the diffusion dynamics of the EGFR can be used as an indicator for drug evaluation. However, because the activity of a group of molecules downstream of the signaling pathway is highly dependent on the multimer formation of the EGFR as well as the phosphorylation of the EGFR itself, the movement of the EGFR alone cannot be used to accurately estimate the downstream effects in the pathway, and the signaling activity of the RTK including the EGFR cannot be adequately evaluated.

Therefore, the present disclosure provides a novel method by which the activity of the RTK can be evaluated.

Solution to Problem

In one aspect, the present disclosure relates to a method for evaluating an activity of a receptor tyrosine kinase (RTK), the method including: a step (A1) for determining diffusion dynamics A1 of the RTK by contacting a target substance with a cell that expresses a labeled receptor tyrosine kinase (RTK) on a cell membrane; a step (B1) for determining diffusion dynamics B1 of the RTK by contacting the target substance with a cell that expresses the labeled receptor tyrosine kinase (RTK) on a cell membrane and a ligand that activates the RTK; a step (C1) for calculating a ratio of the diffusion dynamics A1 of the RTK obtained at the step (A1) and the diffusion dynamics B1 of the RTK obtained at the step (B1), (diffusion dynamics B1 / diffusion dynamics A1); and a step (D1) for evaluating an effect of the target substance on the RTK activity by comparing the ratio of the diffusion dynamics (diffusion dynamics B1 / diffusion dynamics A1) obtained at the step (C1) with a threshold; and at the step (D1), the ratio of the diffusion dynamics (diffusion dynamics B1 / diffusion dynamics A1) obtained at the step (C1) being equal to or greater than the threshold can indicate that the target substance has an effect on a molecular function of the RTK.

In another aspect, the present disclosure relates to a method for evaluating an activity of a receptor tyrosine kinase (RTK), the method including: a step (A2) for determining diffusion dynamics A2 of the RTK without contacting a target substance with a cell that expresses a labeled receptor tyrosine kinase (RTK) on a cell membrane, a step (B2) for determining diffusion dynamics B2 of the RTK by contacting the target substance with a cell that expresses the labeled receptor tyrosine kinase (RTK) on a cell membrane, a step (C2) for calculating a ratio of the diffusion dynamics A2 of the RTK obtained at the step (A2) and the diffusion dynamics B2 of the RTK obtained at the step (B2) (diffusion dynamics B2 / diffusion dynamics A2) or calculating a difference between the diffusion dynamics B2 and the diffusion dynamics A2 (diffusion dynamics B2 - diffusion dynamics A2), a step (E2) for determining diffusion dynamics E2 of the RTK without contacting the target substance with a cell that expresses the labeled receptor tyrosine kinase (RTK) on a cell membrane, a step (F2) for determining diffusion dynamics F2 of the RTK by contacting a solution not containing the target substance with the cell of the step (E2), a step (G2) for calculating a ratio of the diffusion dynamics E2 of the RTK obtained at the step (E2) and the diffusion dynamics F2 of the RTK obtained at the step (F2) (diffusion dynamics F2 / diffusion dynamics E2), or calculating a difference between the diffusion dynamics E2 and the diffusion dynamics F2 (diffusion dynamics F2 - diffusion dynamics E2), and a step (D2) for evaluating an RTK activity of the target substance by comparing a threshold with an absolute value of a difference between the ratios of the diffusion dynamics obtained at the step (C2) (diffusion dynamics B2 / diffusion dynamics A2) and the diffusion dynamics ratio obtained at the step (G2) (diffusion dynamics F2 / diffusion dynamics E2) ([diffusion dynamics B2 / diffusion dynamics A2] - [diffusion dynamics F2 / diffusion dynamics E2]), or with an absolute value of the difference between the difference of the diffusion dynamics obtained at the step (C2) (diffusion dynamics B2 - diffusion dynamics A2) and the difference of the diffusion dynamics obtained at the step (G2) (diffusion dynamics F2 - diffusion dynamics E2) ([diffusion dynamics B2 - diffusion dynamics A2] - [diffusion dynamics F2 - diffusion dynamics E2]); and at the step (D2), the absolute value of the difference between the ratios of the diffusion dynamics ([diffusion dynamics B2 / diffusion dynamics A2] - [diffusion dynamics F2 / diffusion dynamics E2]) or the absolute value of the difference in the diffusion dynamics ([diffusion dynamics B2 - diffusion dynamics A2] - [diffusion dynamics F2 - diffusion dynamics E2]) obtained at the step (G2) being equal to or greater than the threshold can indicate that the target substance has an effect on the RTK's molecular function not limited to a phosphorylation activity.

In another aspect, the present disclosure relates to a method for determining a quantified value of a molecular function and/or an activity of a receptor tyrosine kinase (RTK), and/or a 50% effective concentration ($EC_{50}$) of a target substance or a ligand on the molecular function and/or the activity of the RTK caused by the ligand. The method includes: a step (A3) for determining diffusion dynamics A3 of the RTK without contacting the target substance and the ligand that activates the RTK with a cell that expresses a labeled receptor tyrosine kinase (RTK) on a cell membrane, a step (B3) for determining diffusion dynamics B3 of the RTK by contacting the target substance with a cell that expresses the labeled receptor tyrosine kinase (RTK) on a cell membrane and the ligand that activates the RTK, a step (C3) for calculating a ratio of the diffusion dynamics A3 of the RTK obtained at the step (A3) and the diffusion dynamics B3 of the RTK obtained at the step (B3) (diffusion dynamics B3 / diffusion dynamics A3), and a step (D3) for determining a quantified value of the RTK's molecular function and/or activity caused by the target substance or the ligand, and/or determining the 50% effective concentration ($EC_{50}$) of the target substance or the ligand on the RTK's molecular function and/or activity, using the ratio of the diffusion dynamics (diffusion dynamics B3 / diffusion dynamics A3) obtained at the step (C3).

In still another aspect, the present disclosure relates to a method for evaluating an RTK activity. The method includes: a step (A4) for contacting a target substance with a cell that expresses a labeled receptor tyrosine kinase (RTK) on a cell membrane, a step (B4) for contacting the target substance and a ligand that activates the RTK with a cell that expresses the labeled receptor tyrosine kinase (RTK) on a cell membrane, a step (C4) for quantifying distributions of the degree of RTK multimerization at the step (A4) and the step (B4), and a step (D4) for evaluating the RTK activity of the target substance by comparing the value obtained at the step (C4) with a threshold, and the difference between the value obtained at the step (C4) and the threshold being equal to or greater than a predetermined value at the step (D4) can indicate that the target substance has an effect on a molecular function of the RTK.

In still another aspect, the present disclosure relates to a method for evaluating an activity of a receptor tyrosine kinase (RTK). The method includes: a step (A5) for contacting a target substance with a cell that expresses a labeled receptor tyrosine kinase (RTK) on a cell membrane, a step (B5) for quantifying a distribution of the degree of RTK multimerization before and after the contact of the target substance, and a step (C5) for evaluating the target substance on the RTK's molecular function and/or activity by comparing the value obtained at the step (B5) with a threshold, in which the difference between the value obtained at the step (B5) and the threshold being equal to or greater than a predetermined value at the step (C5) can indicate that the target substance has an effect on the RTK's molecular function and/or activity.

In still another aspect, the present disclosure relates to a method for screening a candidate substance that inhibits a molecular function and/or an activity targeting a receptor tyrosine kinase (RTK) (antagonist candidate) or a candidate substance that activates the RTK (agonist candidate). The method includes evaluating a target substance by the evaluation method of the RTK of the present disclosure, and determining by a quantified value of the RTK's molecular function and/or activity of the present disclosure and/or by a determination method of a 50% effective concentration ($EC_{50}$) of a phosphorylation of the RTK caused by a ligand.

Effects of Invention

In one aspect, the present disclosure provides a novel method with which the RTK activity can be evaluated.

Description of Embodiments

In the course of further research to provide a novel method to evaluate the RTK activity, the inventors of the present invention have found that there is a correlation between the diffusion movement of the EGFR obtained by a single-molecule analysis and the amount of the EGFR's autophosphorylation, and that the amount of the EGFR's autophosphorylation can be quantitatively evaluated based on such diffusion movement.

An RTK is the receptor that acts on a cell membrane and is an important molecule involving in various intracellular signaling pathways such as a cell proliferation. When a ligand is bound to an RTK dimer, the autophosphorylation of the RTK takes place, and then an extracellular signal is transduced into a cell through various reaction processes, such as reduction of an RTK diffusion movement, a multimer formation, and an incorporation of the dimer or multimer into the cell.

The inventors of the present invention attempted to trace the diffusion movement of the EGFR and changes in multimer formation during the series of signaling processes from an EGF binding to cellular uptake at a single-molecule level by fluorescent labeling of the EGFR in a cell and performing single-molecule analysis to visualize the EGFR at the single-molecule level in a cell. During the course of these research processes, it was found that the diffusion movement (diffusion dynamics) of the EGFR obtained from the single-molecule fluorescence moving image of a fluorescently labeled EGFR is correlated with the amount of autophosphorylation of the EGFR. Furthermore, by reading the changes of the multimer formation from the fluorescence intensity of each fluorescently labeled EGFR obtained by the single-molecule analysis, it was found that not only the autophosphorylation level of the EGFR but also the signaling activity of the EGFR can be quantitatively evaluated in a cell. Based on these findings, the inventors of the present invention have found a novel quantitative drug evaluation method that allows exploring a compound and/or a drug from a novel perspective.

Specifically, a receptor diffusion movement (diffusion dynamics) obtained from a single-molecule fluorescent image decreases with the concentration of a ligand (e.g., EGF), which is proportional to the increase in phosphorylation of a receptor (e.g., EGFR). In addition, the receptor multimer that is formed upon a ligand stimulation is the starting point for the downstream signaling. Therefore, the receptor autophosphorylation can be quantitatively estimated by measuring the receptor diffusion movement. Furthermore, by measuring, before and after the addition of the ligand, the diffusion movement of the cell having the target substance contacted and calculating the diffusion dynamics of the receptor (EGFR), and obtaining the ratio of the obtained diffusion dynamics (= [diffusion dynamics after ligand addition] / [diffusion dynamics before ligand addition]), it was found that the movement of only the molecule (receptor) pertinent to the activation that may be generated by the ligand can be read more accurately.

In addition, it was also found that a distribution of the fluorescence intensity obtained by the single-molecule analysis can be used as a characteristic value of the multimer formation of the RTK such as EGFR, and further found that by using a Kullback-Leibler divergence (KL divergence) as the quantitative evaluation method of the frequency distribution of the fluorescence intensity, a novel drug evaluation method that allows searching for a compound/drug that has been difficult to be detected by existing methods can be realized.

In the present disclosure, the RTKs are membrane proteins for transmembrane locally present on a cell membrane and usually has a structure in which the ligand binding site located in an extracellular region and a tyrosine kinase region (phosphotransferase region to a tyrosine residue) in the intracytoplasmic region are linked by a single transmembrane region. It is also known that the RTKs have a three-dimensional structure that is very similar to each other. Today, the RTKs are classified into 20 subfamilies. In one or more embodiments, the RTK subfamily include, but are not limited to, an EGFR family, an FGFR (Fibroblast Growth Factor Receptor) family, a PDGFR (Platelet-Derived Growth Factor Receptor) family, a VEGFR (Vascular Endothelial Growth Factor Receptor) family, and an RET (Rearranged During Transection) family.

The "RTK activity" in the present disclosure includes, in one or more embodiments, the (auto)phosphorylation activity of the RTK.

The "molecular function of RTK (RTK's molecular function)" in the present disclosure may include, in one or more embodiments, an activation of the RTK, a conformational change in an extracellular region of the RTK, a conformational change in an intracellular region of the RTK, a changes of the RTK's interaction with the cell membrane, a formation of a dimer or multimer of the RTK, and an internalization of the RTK. In one or more embodiments, the RTK's molecular function may include a cell proliferation, differentiation, survival, metabolism and/or migration, and physiological response, which may be regulated by the signaling that occurs upon the change in the RTK described above.

In one or more embodiments, "influencing the molecular function of the RTK" means having an antagonistic effect on the molecular function of the RTK or having an operative effect on the molecular function of the RTK. Thus, in one or more embodiments, the evaluation method in the present disclosure may be used to search for an active ingredient of the RTK agonist or antagonist.

"To evaluate the RTK activity" in the present disclosure means to evaluate whether or not the target substance to be evaluated has an effect on the RTK's molecular function and/or to evaluate the extent to which the target substance has an effect on the RTK's molecular function. In one or more embodiments, the evaluation of the RTK activity includes quantitatively evaluating the mobility pertinent to the RTK's (auto)phosphorylation, quantitatively evaluating the formation of multimer of the RTK, quantitatively evaluating the degree of RTK multimerization, evaluating the changes in the multimer that can be formed or has been formed, and quantitatively evaluating a signaling activity.

In one or more embodiments, the RTK in the present disclosure includes, but is not limited to, EGFR, MER (Receptor Tyrosine Kinase Mer RTK), ERBB2 (Erb-B2 receptor tyrosine kinase 2), ERBB4 (Erb-B4 receptor tyrosine kinase 4), FGFR1, FGFR2, FGFR3, INSR (Insulin Receptor), HER3 (Human Epidermal growth factor Receptor 3)/ERBB3, MUSK (Muscle-Specific Kinase), KIT (KIT Proto-Oncogene), PDGFR$\alpha$, PDGFR$\beta$, RET, ROR2 (Receptor Tyrosine Kinase-like orphan Receptor 2), NTRK1 (Neurotrophic Receptor Tyrosine Kinase 1), FLT3 (Fms-like tyrosine kinasE4), FLT4, FLK2 (Fetal Liver Kinase 2), and TRKA (Tropomyosin Receptor Kinase A).

In one or more embodiments, the ligand that activates the RTK in the present disclosure may include known ligands for the RTK described above. In one or more embodiments, examples of the ligand for EGFR include EGF, TGF-$\alpha$ (transforming growth factor alpha), AR (amphiregulin), BTC (betacellulin), and HB -EGF (heparin-binding EGF-like growth factor). In one or more embodiments, examples of the common ligand for ERBB3 and ERBB4 include HRG (heregulin, or NRG (neuregulin) 1) and NRG2. In one or more embodiments, examples of the ligand for ERBB4 include NRG3, NRG4, BTC, and HB-EGF.

The "diffusion dynamics" in the present disclosure is one of the indicators to evaluate the molecular dynamics (lateral diffusion) of the RTK molecule that may occur when the RTK is stimulated by the ligand or other stimuli. In one or more embodiments, the diffusion dynamics may be determined based on the RTK positional information. That is, from the positional information, in one or more embodiments, a speed, a diffusion range, and the like of the RTK molecule, which reflect the RTK's molecular structure and cell membrane environment, can be calculated.

In one or more embodiments, the "diffusion dynamics" in the present disclosure may be determined based on the positional information obtained by single-molecule imaging of a labeled RTK. In one or more embodiments, as for the indicator of the diffusion dynamics determined based on the positional information, a mean square displacement (MSD) and/or a diffusion coefficient ($D_{Avg}$) may be used. In one or more embodiments, the mean square displacement (MSD) and the diffusion coefficient ($D_{Avg}$) may be calculated by the methods to be described later and in Examples.

The "distribution of degree of multimerization" in the present disclosure is one of the indicators used to evaluate the RTK signaling. The RTK changes its association state in response to a stimulation such as a ligand; therefore, for example, monomers may aggregate to form a dimer or a multimer, or a dimer or a multimer may change to monomers.

In one or more embodiments, the "distribution of the degree of multimerization" in the present disclosure may be determined based on the luminance information obtained by single-molecule imaging of the labeled RTK. In one or more embodiments, luminance is the luminance of a labeling substance that labels the RTK. In one or more embodiments, the degree of aggregation (multimerization) of the RTK molecule may be obtained from the luminance information. In one or more embodiments, examples of the method for quantifying the distribution of the degree of RTK multimerization before and after the contact of a ligand or a target substance may include a method for quantifying, using the KL divergence, the difference between the distribution of the degree of multimerization before the contact of the ligand or with the target substance and the distribution of the degree of multimerization after the contact of the ligand or with the target substance. In one or more embodiments, the value of the KL divergence may be calculated by the method to be described later and in Examples.

In the present disclosure, the "single-molecule imaging" refers to visualization of individual molecules by labeling a biomolecule such as a protein with a labeling substance such as a fluorescent protein, and also refers to the technique for visualizing the behavior of individual molecules (single molecules) within a cell. With single-molecule imaging, the labeled molecule may be tracked at the level of a single molecule. In one or more embodiments, single-molecule imaging may also be referred to as a cellular single-molecule imaging. In one or more embodiments, single-molecule imaging may be performed using a total internal reflection fluorescence microscopy (TIRFM). In one or more embodiments not particularly limited, single-molecule imaging is performed, for example, under a fluorescence microscope using a total reflection illumination to excite a fluorescent label attached to the RTK on a cell membrane before and after the contact of the target substance thereby acquiring information such as the positional and luminance information of the bright spot based on the fluorescent label, and further acquiring information on the trajectory of each bright spot by connecting the bright spots most closely located in the time-series images under a certain threshold. In one or more embodiments, single-molecule imaging may be performed using a multi-well plate, such as a 96-well plate. In one or more embodiments, single-molecule imaging may be performed in accordance with the following disclosures: M. Yanagawa et al., Science Signaling, 2018 Sep 18, Vol. 11, Issue 548, eaao1917, WO 2019/159627, and Japanese Patent Application Laid-open No. 2021- 29165, among other publications. In one or more embodiments, single-molecule imaging may be performed using an AiSIS (Automated in-cell Single-molecule Imaging System), which is a microscope system developed by the inventors of the present invention. The contents of these references are incorporated herein by reference, so that they constitute part of the present disclosure.

The term "labeled RTK" in the present disclosure refers to the RTK that is labeled by a labeling substance. In one or more embodiments, examples of the labeling substance include a fluorescent substance. In one or more embodiments, examples of the labeled RTK include the RTK that is labeled with a fluorescent substance. In one or more embodiments, the examples of the fluorescent substance may include fluorescent dyes such as fluorescein, rhodamine, Texas red, tetramethylrhodamine, carboxyrhodamine, and phycoerythrin. In one or more embodiments, the fluorescent substance may be attached to any position of the RTK, for example, to the C-terminal of the RTK. Alternatively, in one or more embodiments, a fluorescent ligand may be covalently attached by attaching a tag sequence (e.g., HaloTag, SNAP-tag, and CLIP-tag) to express in the cell as a fusion protein having the fluorescent protein attached. In one or more embodiments, when using the tag sequence to which the fluorescent ligand is covalently bonded or the fusion protein with the fluorescent protein, it is preferable to express on a cell membrane the fusion protein containing the RTK and the fluorescent protein or the tag sequence from the N-terminal to C-terminal direction. When fluorescently labeling at the C-terminal, the fluorescent protein or the tag sequence in the expressing fusion protein is located on the inner side of the cell membrane.

In one or more embodiments, the fluorescent labeling using the fluorescent protein may be mentioned as the typical labeling. In one or more embodiments, examples of the fluorescent protein include a GFP.

There is no particular restriction in the cell that expresses the RTK on a cell membrane as far as it is capable of expressing the RTK on a cell membrane; in one or more embodiments, the examples of the cell may include a cell originated from eukaryotic, preferably a cell originated from vertebrate. In one or more embodiments, the examples of the cell that expresses the labeled RTK on a cell membrane may include a cell that expresses the fluorescently labeled RTK and a CHO cell that expresses the fluorescently labeled RTK.

In one or more embodiments, the RTK may be expressed on a cell membrane using a known technique. In one or more embodiments, the cell that expresses the labeled RTK on a cell membrane may be obtained by introducing a gene encoding the fusion protein containing the RTK and the fluorescent protein or the tag sequence, or an expression vector containing such a gene, into a cell to express the fusion protein on the cell membrane.

There is no particular restriction in the target substances; in one or more embodiments, the examples thereof may include a nucleic acid, a peptide, a protein, a synthetic compound, a supernatant of a microbial culture, a plant-originated natural component, a plant extract, and an animal tissue extract.

[First method for evaluating the RTK activity]

In one aspect, the present disclosure relates to a method for evaluating an RTK activity by a target substance using a ratio of the diffusion dynamics of the RTK (first method for evaluating the RTK activity). A first method for evaluating an RTK activity in the present disclosure relates to the evaluation method of the RTK activity, the method including: a step (A1) for determining diffusion dynamics A1 of the RTK by contacting a target substance with a cell that expresses a labeled RTK on a cell membrane, a step (B1) for determining diffusion dynamics B1 of the RTK by contacting the target substance and a ligand that activates the RTK with a cell that expresses the labeled RTK on a cell membrane, a step (C1) for calculating a ratio of the diffusion dynamics A1 of the RTK obtained at the step (A1) and the diffusion dynamics B1 of the RTK obtained at the step (B1), (diffusion dynamics B1 / diffusion dynamics A1), and a step (D1) for evaluating an effect of the target substance on the RTK activity by comparing the ratio of the diffusion dynamics obtained at the step (C1) with a threshold; and at the step (D1), the ratio of the diffusion dynamics obtained at the step (C1) being equal to or greater than the threshold can indicate that the target substance has an effect on a molecular function of the RTK.

With the first method for evaluating the RTK activity of the present disclosure, the ratio of the diffusion dynamics of the RTK before and after the ligand contact (diffusion dynamics B1 / diffusion dynamics A1) in the cell having the target substance contacted is used to evaluate the RTK activity; thus, in one or more embodiments, only the activation-related RTK's movement that may be caused by the ligand can be read more accurately. With the first method for evaluating the RTK activity of the present disclosure, in one or more embodiments, the effect of the target substance on the activation of RTK due to the ligand binding may be accurately evaluated. In one or more embodiments, the first method for evaluating the RTK activity in the present disclosure is to evaluate whether or not the target substance has the ability to inhibit the autophosphorylation of the RTK. In one or more embodiments, the first method for evaluating the RTK activity of the present disclosure may be used to evaluate whether or not the target substance exhibits an antagonistic activity against the RTK or whether or not the target substance can be an antagonist of the RTK.

[Step (A1) and step (B1)]

The first method for evaluating the RTK activity of the present disclosure includes the step (A1) for determining the diffusion dynamics A1 of the RTK by contacting the target substance with the cell that expresses the labeled RTK on a cell membrane, and the step (B1) for determining the diffusion dynamics B1 of the RTK by contacting the target substance and the ligand that activates the RTK with a cell that expresses the labeled RTK on a cell membrane.

The step (A1) is the step for determining the diffusion dynamics A1 of the RTK in a cell in the presence of the target substance and in the absence of the ligand, and the step (B1) is the step for determining the diffusion dynamics B1 of the RTK in a cell in the presence of the target substance contacted at the step (A1) and the ligand not contacted at the step (A1).

In one or more embodiments, the cell at the step (B1) may be different from the cell having contacted with the target substance at the step (A1), or may be the cell having contacted with the target substance at the step (A1). In one or more embodiments, the step (B1) of the first method for evaluating the RTK activity of the present disclosure may include determining the diffusion dynamics B1 of the RTK by contacting the ligand that activates the RTK with the cell having contacted with the target substance at the step (A1).

In one or more embodiments, the step (B1) may involve contacting the target substance and the ligand with the cell that expresses the labeled RTK on a cell membrane at the same time or separately.

In the present disclosure, the term "contact" refers to bringing the target substance and the ligand into a state in which they can affect the RTK activity, preferably in a state in which the target substance and/or the ligand can bind to the RTK.

In one or more embodiments, the contact of the target substance and the ligand that activates the RTK with the cell that expresses the RTK on a cell membrane may involve contacting the target substance and the ligand with the cell at the same time, contacting the target substance with the cell and then contacting the ligand with the cell, or contacting the ligand with the cell and then contacting the target substance with this cell. In one or more embodiments, there is no particular restriction in the concentrations of the target substance and of the ligand in contact with the cell; they may be determined as appropriate.

In one or more embodiments, the target substance and the ligand that activates the RTK may be brought into contact with the cell under two or more different concentration conditions or under one concentration condition. In one or more embodiments, the concentration of the target substance may be fixed and the concentration of the ligand may be varied to different concentration conditions; on the other hand, the concentration of the ligand may be fixed and the concentration of the target substance may be varied to different concentration conditions. In one or more embodiments, the different concentration conditions include two or more multiple conditions with different orders.

The contact time may be set as desired, ranging from 10 seconds to 3 hours in one or more embodiments. In one or more embodiments, the contact time is 30 seconds or more, 1 minute or more, 5 minutes or more, or 10 minutes or more. In one or more embodiments, the contact time is 2 hours or less, 1 hour or less, or 30 minutes or less.

In one or more embodiments, the contact of the target substance and the ligand with the cell may be performed, for example, by mixing them, or by dropping the target substance and/or the ligand onto the cell. In one or more embodiments, the target substance and/or the ligand may be mixed with the cell as it is contained in a solution such as a buffer or a culture medium, or may be dropped into a buffer or a culture medium containing the cell. In one or more embodiments, the examples of the buffer may include phosphoric acid, Bicine (N,N-bis(2-hydroxyethyl)glycine), BES (N,N-bis(2-hydroxyethyl)-2-aminoethanesulfonic acid), MOPS (3-morpholinopropanesulfonic acid), TES (N-tris(hydroxymethyl)methyl-2-aminoethanesulfonic acid), HEPES (2-[4-(2-hydroxyethyl)-1-piperazinyl]ethanesulfonic acid), TRICINE (N-[tris(hydroxymethyl)methyl]glycine), PIPES (piperazine-1,4-bis(2-ethanesulfonic acid)), and POPSO (piperazine-1,4-bis(2-hydroxy-3-propanesulfonic acid) dehydrate). In one or more embodiments, the examples of the medium may include Dulbecco's Modified Eagle's Medium (DMEM), Ham's Nutrient Mixture F12, DMEM/F12 Medium, McCoy's 5A Medium, Eagle's Minimum Essential Medium (EMEM), alpha Modified Eagle's Minimum Essential Medium (αMEM), MEM Medium (Minimum Essential Medium), and RPMI1640 Medium.

In one or more embodiments, the diffusion dynamics at the step (A1) and the step (B1) may be determined by calculating the mean square displacement (MSD) of the RTK trajectory (positional information) in each cell or the diffusion coefficient ($D_{Avg}$). In one or more embodiments, the trajectory of the RTK in the cell includes the positional information obtained by single-molecule imaging of the labeled RTK.

In one or more embodiments, the mean square displacement (MSD) may be calculated from the following formula based on the trajectory (positional information) of the RTK (molecule) obtained by single-molecule imaging.

[Math. 1]

$$\mathrm{MSD}(n \cdot \delta t) = \frac{1}{N-1-n} \sum_{j=1}^{N-1-n} \{[x(j \cdot \delta t + n \cdot \delta t) - x(j \cdot \delta t)]^2 + [y(j \cdot \delta t + n \cdot \delta t) - y(j \cdot \delta t)]^2\}$$

In the above formula, N represents the total number of frames in the trajectory, n represents the number of frames (number of interval frames), $\delta t$ represents the frame rate (time interval between frames), j represents the initial frame, and (x, y) represents the molecular position. The MSD may be calculated by referring to Examples, as well as the articles, M. Yasui et al., nature commun, 2018, Vol. 9, 3061 and M. Hiroshima et al., Microscopy, 2020, Vol. 69, No. 2 69-78, among other articles. The contents of these references are incorporated herein by reference, so that they constitute part of the present disclosure.

In one or more embodiments, the diffusion coefficient ($D_{Avg}$) may be calculated from the following formula based on the two-dimensional diffusion equation.

[Math. 2]

$$D_{Avg}(n \cdot \delta t) = \frac{1}{M} \sum_{j=1}^{M} \frac{MSD_j (n \cdot \delta t)}{4n \cdot \delta t}$$

In the above formula, $MSD_j$ represents the MSD of the jth trajectory and M is the number of total trajectories.

In one or more embodiments, in order to determine the diffusion dynamics, the step (A1) and the step (B1) may include obtaining the positional information of the labeled RTK that expresses on a cell. In one or more embodiments, single-molecule imaging, preferably fluorescent single-molecule imaging, may be mentioned as the method for obtaining the RTK positional information. In one or more embodiments not particularly restricted, the RTK positional information may be obtained by measuring the cell in which the labeled RTK is expressed by means of single-molecule imaging thereby imaging (observing) this label for a prescribed time.

In one or more embodiments of the first method for evaluating the RTK activity in the present disclosure, the step (A1) may include contacting the target substance with the cell that expresses the labeled RTK on a cell membrane, measuring the cell having contacted with the target substance by single-molecule imaging, and determining the diffusion dynamics A1 of the RTK using the positional information obtained by single-molecule imaging; and the step (B1) may include contacting the ligand that activates the RTK with the cell having contacted with the target substance at the step (A1), measuring the cell having contacted with the ligand by single-molecule imaging, and determining the diffusion dynamics B1 of the RTK using the positional information obtained by single-molecule imaging. In one or more embodiments, the measurement of the cell by single-molecule imaging includes obtaining the positional information of the RTK in the cell.

[Step (C1)]

The first method for evaluating the RTK activity of the present disclosure includes the step (C1) for calculating a ratio of the diffusion dynamics A1 of the RTK obtained at the step (A1) and the diffusion dynamics B1 of the RTK obtained at the step (B1) (diffusion dynamics B1 / diffusion dynamics A1). In one or more embodiments, the ratio of the diffusion dynamics calculated at the step (C1) is the ratio of the mean square displacements (MSD) or the ratio of the diffusion coefficients; it is preferably the ratio of the MSD. In one or more embodiments, the ratio of MSD may be determined by the method to be described in Examples.

[Step (D1)]

The first method for evaluating the RTK activity of the present disclosure includes the step (D1) for evaluating the effect of the target substance on the RTK activity by comparing the ratio of the diffusion dynamics obtained at the step (C1) with a threshold. When the ratio of the diffusion dynamics (diffusion dynamics B1 / diffusion dynamics A1) obtained at the step (C1) is equal to or greater than the threshold, it can evaluate that the target substance in contact with the cell has an effect on the molecular function of the RTK. In one or more embodiments, the evaluation of the effect on the molecular function of the RTK includes the evaluation of the ability of the target substance to inhibit the autophosphorylation of the RTK. In one or more embodiments, the evaluation of the effect on the molecular function of the RTK includes evaluating that the target substance exhibits an antagonistic effect on the RTK or can be an antagonist of the RTK.

In one or more embodiments, the threshold may be determined based on the ratio of the diffusion dynamics obtained under the control condition (without contact of the target substance). In one or more embodiments not particularly restricted, the threshold may be calculated from the following formula.

[Threshold] = [average ratio of diffusion dynamics in control] + 3 × standard deviation

In one or more embodiments, the first method for evaluating the RTK activity of the present disclosure may further include a step for determining the threshold. In one or more embodiments, the threshold may be determined using the ratio of the diffusion dynamics of the RTK before and after the contact of the ligand in a cell in the absence of the target substance.

In one or more embodiments, the process for determining the threshold may include the step (E1) for determining the diffusion dynamics E1 of the RTK without contacting the ligand that activates the RTK with a cell that expresses the RTK on a cell membrane, the step (F1) for determining the diffusion dynamics F1 of the RTK by contacting the ligand that activates the RTK with a cell that expresses the RTK on a cell membrane, and the step (G1) for calculating the ratio of the diffusion dynamics E1 of the RTK obtained at the step (E1) and the diffusion dynamics F1 of the RTK obtained at the step (F1) (diffusion dynamics F1 / diffusion dynamics E1), in which the threshold is determined based on the ratio of the diffusion dynamics (diffusion dynamics F1 / diffusion dynamics E1) obtained at the step (G1). In one or more embodiments, at the step (E1) and the step (F1), the target substance is not brought into contact with the cell. The contact with the cell and the determination of the diffusion dynamics at the step (E1) and the step (F1) may be performed in the same way as the step (A1) and the step (B1) described above. The calculation of the ratio of the diffusion dynamics at the step (G1) may be performed in the same way as at the step (C1) described above.

In one or more embodiments not particularly restricted, the threshold may be determined from the following formula using the average value of the ratio of the diffusion dynamics (diffusion dynamics F1 / diffusion dynamics E1) obtained at the step (G1) and its standard deviation.

[Threshold] = average value of [diffusion dynamics F1 / diffusion dynamics E1] + 3 × standard deviation

In one or more embodiments, the first method for evaluating the RTK activity of the present disclosure may include the evaluation that the difference between the ratio of the diffusion dynamics obtained at the step (C1) (diffusion dynamics B1 / diffusion dynamics A1) and the ratio of the diffusion dynamics obtained at the step (G1) (diffusion dynamics F1 / diffusion dynamics E1) ([diffusion dynamics B1 / diffusion dynamics A1] - [diffusion dynamics F1 / diffusion dynamics E1]) exceeding a predetermined value can indicate that the target substance has an effect on the molecular function of the RTK.

Therefore, in one or more embodiments, the step (D1) may include calculating the difference between the ratio of the diffusion dynamics obtained at the step (C1) (diffusion dynamics B1 / diffusion dynamics A1) and the ratio of the diffusion dynamics obtained at the step (G1) (diffusion dynamics F1 / diffusion dynamics E1), ([diffusion dynamics B1 / diffusion dynamics A1] - [diffusion dynamics F1 / diffusion dynamics E1]), comparing the obtained value (difference between the ratios of the diffusion dynamics) with a predetermined value (threshold), and evaluating that as a result of the comparison the difference between the ratios of the diffusion dynamics obtained ([diffusion dynamics B1 / diffusion dynamics A1] - [diffusion dynamics F1 / diffusion dynamics E1]) exceeding the threshold or a predetermined value can indicate that the target substance has an effect on the molecular function of the RTK.

In one or more embodiments, "exceeding a predetermined value" in the present disclosure means that the number is equal to or greater than a significant difference.

[Second method for evaluating the RTK activity]

In another aspect, the present disclosure relates to a method for evaluating an RTK activity by a target substance using a ratio or a difference of diffusion dynamics of the RTK (second method for evaluating the RTK activity). The second method for evaluating the RTK activity in another aspect of the present disclosure relates to a method for evaluating an RTK activity, the method including: a step (A2) for determining diffusion dynamics A2 of the RTK without contacting a target substance with a cell that expresses a labeled RTK on a cell membrane, a step (B2) for determining diffusion dynamics B2 of the RTK by contacting the target substance with a cell that expresses the labeled RTK on a cell membrane, a step (C2) for calculating a ratio of the diffusion dynamics A2 of the RTK obtained at the step (A2) and the diffusion dynamics B2 of the RTK obtained at the step (B2) (diffusion dynamics B2 / diffusion dynamics A2) or a difference between the diffusion dynamics B2 and the diffusion dynamics A2 (diffusion dynamics B2 - diffusion dynamics A2), a step (E2) for determining diffusion dynamics E2 of the RTK without contacting the target substance with a cell that expresses the labeled RTK on a cell membrane, a step (F2) for determining diffusion dynamics F2 of the RTK by contacting a solution not containing the target substance with the cell of the step (E2), a step (G2) for calculating a ratio of the diffusion dynamics E2 of the RTK obtained at the step (E2) and the diffusion dynamics F2 of the RTK obtained at the step (F2) (diffusion dynamics F2 / diffusion dynamics E2), or a difference between the diffusion dynamics E2 and the diffusion dynamics F2 (diffusion dynamics F2 - diffusion dynamics E2), and a step (D2) for evaluating the RTK activity of the target substance by comparing an absolute value of the difference between the ratio of the diffusion dynamics obtained at the step (C2) (diffusion dynamics B2 / diffusion dynamics A2) and the ratio of the diffusion dynamics obtained at the step (G2) (diffusion dynamics F2 / diffusion dynamics E2), ([diffusion dynamics B2 / diffusion dynamics A2] - [diffusion dynamics F2 / diffusion dynamics E2]), or an absolute value of the difference between the diffusion dynamics difference obtained at the step (C2) ([diffusion dynamics B2 - diffusion dynamics A2]) and the diffusion dynamics difference ([diffusion dynamics F2 - diffusion dynamics E2 ) obtained at the step (G2) ([diffusion dynamics B2 - diffusion dynamics A2] - [diffusion dynamics F2 - diffusion dynamics E2]) with a threshold; and at the step (D2), the ratio of the diffusion dynamics or the difference of the diffusion dynamics obtained at the step (C2) being equal to or greater than the threshold can indicate that the target substance has an effect on the molecular function of the RTK not limited to the phosphorylation activity.

With the second method for evaluating the RTK activity of the present disclosure, in one or more embodiments, only the movement of the RTK by the target substance can be read more accurately, because the ratio of the diffusion dynamics of the RTK before and after the contact of the target substance is used to evaluate the RTK activity. In one or more embodiments, the second method for evaluating the RTK activity of the present disclosure may allow accurate evaluation of the effect of the target substance on the RTK. In one or more embodiments, the second method for evaluating the RTK activity of the present disclosure can be used to evaluate whether or not the target substance can promote the autophosphorylation of the RTK or whether or not the target substance can become an agonist of the RTK. In one or more embodiments, the second method for evaluating the RTK activity of the present disclosure may be used to evaluate whether or not the target substance has the ability to activate the RTK not limited to the phosphorylation activity.

The step (A2) is for determining the diffusion dynamics A2 of the RTK without contacting the target substance with the cell that expresses the labeled RTK on a cell membrane. The step (A2) may be performed in the same way as the step (A1) of the first method for evaluating the RTK activity, except that the cell is not contacted with the target substance and the ligand.

The step (B2) is for determining the diffusion dynamics B2 of the RTK by contacting the target substance with the cell that expresses the labeled RTK on a cell membrane. The step (B2) may be performed in the same way as the step (B1) of the first method for evaluating the RTK activity of the present disclosure, except that the ligand is not contacted. In one or more embodiments, the cell at the step (B2) may be different from the cell used at the step (A2), or may be the cell used at the step (A2). In one or more embodiments, the second method for evaluating the RTK activity of the present disclosure may also include, at the step (B2), determining the diffusion dynamics B2 of the RTK by contacting the target substance with the cell of the step (A2).

The step (C2) includes calculating the ratio of the diffusion dynamics A2 of the RTK obtained at the step (A2) and the diffusion dynamics B2 of the RTK obtained at the step (B2) (diffusion dynamics B2 / diffusion dynamics A2) or the difference between the diffusion dynamics B2 and the diffusion dynamics A2 (diffusion dynamics B2 - diffusion dynamics A2).

In one or more embodiments, the threshold may be determined based on the ratio of the diffusion dynamics or the difference between them obtained under the control condition (without contacting the target substance). In one or more embodiments, the threshold may also be determined based on the ratio or the difference of the diffusion dynamics obtained under a positive control (e.g., with addition of a substance known as the ligand for the RTK to be evaluated).

In one or more embodiments, the threshold may be determined using the ratio of the diffusion dynamics of the RTK before and after the contact of a ligand-free solution (e.g., buffer solution) in the absence of the target substance. The step for determining the threshold may be performed at the step (A2) by contacting only the solution containing neither the target substance nor the ligand that activates RTK (e.g., buffer solution only), without contacting the target substance, with the cell that expresses the RTK on a cell membrane.

The step (E2) to the step (G2) are the steps for determining the threshold, in which the solution that is brought into contact with the cell at the step (F2) contains neither the target substance nor the ligand that activates the RTK. In one or more embodiments, a buffer solution or a culture medium may be used as the solution to be brought into contact with the cell at the step (F2).

The contact with the cell and determination of the diffusion dynamics E2 and the diffusion dynamics F2 at the step (E2) and the step (F2) may be performed in the same way as at the step (A2) and the step (B2) described above. The calculation of the ratio of diffusion dynamics (diffusion dynamics F2 / diffusion dynamics E2) or the difference between the diffusion dynamics (diffusion dynamics F2 - diffusion dynamics E2) at the step (G2) may be performed in the same way as at the step (C2) described above.

The step (D2) includes evaluating the RTK activity of the target substance by comparing the threshold with the absolute value of the difference between the ratio of the diffusion dynamics obtained at the step (C2) (diffusion dynamics B2 / diffusion dynamics A2) and the ratio of the diffusion dynamics obtained at the step (G2) (diffusion dynamics F2 / diffusion dynamics E2), ([diffusion dynamics B2 / diffusion dynamics A2] - [diffusion dynamics F2 / diffusion dynamics E2]), or with the absolute value of the difference between the difference of the diffusion dynamics obtained at the step (C2) (diffusion dynamics B2 - diffusion dynamics A2) and the difference of the diffusion dynamics obtained at the step (G2) (diffusion dynamics F2 - diffusion dynamics E2), ([diffusion dynamics B2 - diffusion dynamics A2] - [diffusion dynamics F2 - diffusion dynamics E2]). The absolute value of the difference between the ratios of the diffusion dynamics ([diffusion dynamics B2 / diffusion dynamics A2] - [diffusion dynamics F2 / diffusion dynamics E2]) or the absolute value of the difference in the diffusion dynamics ([diffusion dynamics B2 - diffusion dynamics A2] - [diffusion dynamics F2 - diffusion dynamics E2]) being equal to or greater than the threshold can indicate that the target substance has an effect on the molecular function of the RTK, which is not limited to the phosphorylation activity. In one or more embodiments, the positive evaluation of the effect on the molecular function of the RTK includes the evaluation that the target substance has the ability to activate the phosphorylation of the RTK and/or to activate the RTK including but not limited to the phosphorylation activity. In one or more embodiments, the positive evaluation of the effect on the molecular function of the RTK includes the evaluation that the target substance exhibits an activation effect on the RTK or can become an agonist of the RTK.

[Method for determining the quantitative value and $EC_{50}$ of RTK's phosphorylation activity]

The inventors of the present invention found that the ratio of the diffusion dynamics of the RTK obtained by the second method for evaluating the RTK activity has a linear relationship with the amount of the phosphorylation of the RTK thereby allowing this relationship to be used for the quantitative evaluation of a drug. Thus, as the other aspect, the present disclosure relates to a method for determining, using the ratio of the diffusion dynamics of the RTK, the quantitative value of the phosphorylation activity of the RTK and/or the 50% effective concentration ($EC_{50}$) of the ligand-induced phosphorylation of the RTK.

The method of the present aspect is for a method for determining a quantitative value of the RTK's phosphorylation activity and an $EC_{50}$ of a ligand-induced RTK phosphorylation. The method includes: a step (A3) for determining diffusion dynamics A3 of the RTK without contacting a target substance and a ligand that activates the RTK with a cell that expresses a labeled RTK on a cell membrane, a step (B3) for determining diffusion dynamics B3 of the RTK by contacting the target substance or a ligand that activates the RTK with a cell that expresses the labeled RTK on a cell membrane, a step (C3) for calculating a ratio of the diffusion dynamics A3 of the RTK obtained at the step (A3) and the diffusion dynamics B3 of the RTK obtained at the step (B3) (diffusion dynamics B3 / diffusion dynamics A3), and a step (D3) for determining, using the diffusion dynamics ratio (diffusion dynamics B3 / diffusion dynamics A3) obtained at the step (C3), a quantitative value of the RTK's molecular function and/or activity due to the target substance or the ligand, and/or a 50% effective concentration ($EC_{50}$) of the target substance or the ligand to the RTK's molecular function and/or activity. In one or more embodiment in the present disclosure, the method for determining the quantitative value refers to a quantification method.

The step (A3) through the step (C3) in the method of the present aspect may be performed in the same way as the step (A2) through the step (C2) in the second method for evaluating the RTK activity in the present disclosure. In one or more embodiments, the activity of the RTK in the present aspect includes the phosphorylation of the RTK.

In one or more embodiments, at the step (D3), the $EC_{50}$ may be determined from the following formula, using an MSD ratio as the diffusion dynamics ratio.

[Math. 3]

$$\mathrm{MSD} = \mathrm{MSD}_{max} - \frac{(\mathrm{MSD}_{max} - \mathrm{MSD}_{min})[L]}{[L] + EC_{50}}$$

MSD: averaged MSD ratio
$\mathrm{MSD}_{max}$: maximum value of the average MSD ratio
$\mathrm{MSD}_{min}$: minimum value of the average MSD ratio
[L]: concentration of the EGF (ligand) in the well used for the MSD value.

The quantitative values of the RTK's molecular function and/or activity and the $EC_{50}$ may be determined with reference to Examples.

[Third method for evaluating RTK activity]

In another aspect, the present disclosure relates to a method for evaluating an RTK activity of a target substance (third method for evaluating the RTK activity) using quantification of the distribution of the degree of RTK multimerization. The third method for evaluating the RTK activity of the present disclosure includes: a step (A4) for contacting a target substance with a cell that expresses a labeled RTK on a cell membrane, a step (B4) for contacting the target substance and a ligand that activates the RTK with a cell that expresses the labeled RTK on a cell membrane, a step (C4) for quantifying distributions of the degree of RTK multimerization at the step (A4) and the step (B4), and a step (D4) for evaluating the RTK activity of the target substance by comparing the value obtained at the step (C4) with a threshold, in which the difference between the value obtained at the step (C4) and the threshold being equal to or greater than a predetermined value at the step (D4) can indicate that the target substance has an effect on a molecular function of the RTK.

With the third method for evaluating the RTK activity of the present disclosure, because the distribution of the degree of RTK multimerization before and after the contact of the ligand is quantified and used to evaluate the activity of RTK, in one or more embodiments, a compound/drug that has an effect on the molecular function of RTK that could not be detected by existing methods can be explored. In one or more embodiments, the third method for evaluating the RTK activity of the present disclosure may be used to evaluate whether or not the target substance has an ability to inhibit RTK multimerization. In one or more embodiments, the third method for evaluating the RTK activity of the present disclosure may also be used to evaluate whether or not the target substance exhibits an antagonistic activity against the RTK or whether or not the target substance can become an antagonist of the RTK.

The step (A4) includes contacting the target substance with the cell that expresses the labeled RTK on a cell membrane. The contact of the target substance with the cell may be performed in the same way as the step (A1) of the first method for evaluating the RTK activity of the present disclosure.

In one or more embodiments, the step (A4) is for obtaining the information on the degree of RTK multimerization in the cell in the presence of the target substance and in the absence of the ligand. In one or more embodiments, the step (A4) may include acquiring the luminance of the labeling substance that labels the RTK in order to obtain the information on the degree of RTK multimerization.

In one or more embodiments, the method for acquiring the luminance of the labeling substance that labels the RTK is the method using single-molecule imaging, preferably fluorescent single-molecule imaging. In one or more embodiments not particularly restricted, the luminance of the labeling substance may be obtained by measuring the cell that expresses the labeled RTK by means of single-molecule imaging thereby imaging (observing) the luminance of the labeling substance for a predetermined time period.

In one or more embodiments, the step (A4) may include contacting the target substance with the cell that expresses the labeled RTK on a cell membrane, performing single-molecule imaging of the cell contacted with the target substance, and/or obtaining the luminance of the labeling substance that labels the RTK in the cell contacted with the target substance.

The step (B4) includes contacting the target substance and the ligand that activates the RTK with the cell that expresses the labeled RTK on a cell membrane. The contact of the target substance and the ligand with the cell may be performed in the same way as the step (B1) of the first method for evaluating the RTK activity of the present disclosure.

In one or more embodiments, the step (B4) is for obtaining the information on the degree of RTK multimerization in the cell in the presence of the target substance and the ligand. In one or more embodiments, the step (B4) may include acquiring the luminance of the labeling substance that labels the RTK in order to obtain the information on the degree of RTK multimerization. Thus, in one or more embodiments, the step (B4) may include contacting the target substance and the ligand with the cell that expresses the labeled RTK on a cell membrane, performing single-molecule imaging of the cell contacted with the target substance, and/or acquiring the luminance of the labeling substance that labels the RTK in the cell contacted with the target substance.

The step (C4) includes quantifying the distribution of the degree of RTK multimerization at the step (A4) and the step (B4), i.e., quantifying the distribution of the degree of RTK multimerization before and after the contact of the ligand in the presence of the target substance. In one or more embodiments, the distribution of the degree of RTK multimerization may be determined as the distribution of the luminance of the labeling substance that labels the RTK or as the distribution of the diffusion coefficient of the labeling substance that labels the RTK. In one or more embodiments, the quantification of the distribution of the degree of the multimerization is preferably performed by the KL divergence. In one or more embodiments, by using the KL divergence for quantification, it is possible to efficiently quantify the difference in the amount of multimer formed before and after the ligand addition.

In one or more embodiments, the quantification using the KL divergence may be performed by creating a probability distribution histogram from the frequency distribution of the luminance (e.g., fluorescence intensity) obtained by single-molecule imaging, and using the resulting histogram based on the following formula.

[Math. 4]

$$KL(p \parallel q) = \int_{-\infty}^{\infty} p(x)\ln\frac{p(x)}{q(x)}\,dx$$

p(x): Frequency in the interval x in the probability distribution of the control (before contact of the ligand or the target substance)

q(x): Frequency in the interval x in the probability distribution after contact of the ligand or the target substance

The step (D4) includes evaluating the RTK activity of the target substance by comparing the value obtained at the step (C4) with the threshold. The difference between the value obtained by quantification at the step (C4) and the threshold being equal to or greater than a predetermined value can indicate that the target substance brought into contact with the cell has an effect on the molecular function of the RTK. In one or more embodiments, the evaluation of the effect on the molecular function of the RTK includes the evaluation of the ability of the target substance to inhibit the autophosphorylation of the RTK. In one or more embodiments, the evaluation of the effect on the molecular function of the RTK includes evaluating that the target substance exhibits an antagonistic effect on the RTK or can be an antagonist of the RTK.

In one or more embodiments, "equal to or greater than a predetermined value" in the present disclosure means that the difference with the threshold is equal to or greater than a significant difference.

In one or more embodiments, the threshold may be determined based on the value (e.g., KLD value) obtained by quantifying the distribution of the degree of RTK multimerization obtained under the control condition (without contact of the target substance).

In one or more embodiments, the third method for evaluating the RTK activity of the present disclosure may further include a step for determining the threshold. In one or more embodiments, the step for determining the threshold may include performing single-molecule imaging of a cell that expresses the labeled RTK on a cell membrane, contacting the ligand that activates the RTK with the cell, performing single-molecule imaging of the cell contacted with the ligand, and quantifying, based on the single-molecule imaging, the distribution of the degree of RTK multimerization before and after the contact of the ligand. The contact with the cell, the quantification of the distribution of the degree of multimerization, and single-molecule imaging may be performed in the same way as the step (B4) and the step (C4) described above.

[Fourth method for evaluating the RTK activity]

In another aspect, the present disclosure relates to a method for evaluating an RTK activity of a target substance (fourth method for evaluating the RTK activity) using quantification of the distribution of the degree of RTK multimerization. The fourth method for evaluating the RTK activity of the present disclosure relates to a method for evaluating an RTK activity. The method includes: a step (A5) for contacting a target substance with a cell that expresses a labeled RTK on a cell membrane, a step (B5) for quantifying distribution of the degree of RTK multimerization before and after the contact of the target substance, and a step (C5) for evaluating the target substance on the RTK's molecular function and/or activity by comparing the value obtained at the step (B5) with a threshold and the difference between the value obtained at the step (B5) and the threshold being equal to or greater than a predetermined value at the step (C5) can indicate that the target substance has an effect on the RTK's molecular function and/or activity.

With the fourth method for evaluating the RTK activity of the present disclosure, because the distribution of the degree of RTK multimerization before and after the contact of the target substance is quantified and it is used to evaluate the RTK activity, in one or more embodiments, a compound/drug that has an effect on the RTK's molecular function that cannot be detected by an existing method can be explored. In one or more embodiments, the fourth method for evaluating the RTK activity of the present disclosure may be used to evaluate whether or not the target substance has an ability to promote the multimerization of the RTK. In one or more embodiments, the fourth method for evaluating the RTK activity of the present disclosure may be used to evaluate whether or not the target substance has the ability to activate the RTK not limited to the phosphorylation activity.

The step (A5) includes contacting the target substance with the cell that expresses the labeled RTK on a cell membrane. The step (A5) may be performed in the same way as the step (A2) of the second method for evaluating the RTK activity of the present disclosure, except that the cell is contacted with the target substance but not with the ligand.

The step (B5) includes quantifying the distribution of the degree of RTK multimerization before and after the contact of the target substance at the step (A5). The step (B5) may be performed in the same way as the step (B2) in the second method for evaluating the RTK activity of the present disclosure, except that the distributions of the degrees of RTK multimerization before and after the contact of the target substance are quantified.

The step (C5) includes evaluating the RTK activity of the target substance by comparing the value obtained at the step (B5) with the threshold. The difference between the value obtained at the step (B5) and the threshold being equal to or greater than a predetermined value can indicate that the target substance brought into contact with the cell has an effect on the molecular function of the RTK. In one or more embodiments, the positive evaluation of the effect on the molecular function of the RTK includes the evaluation that the target substance has the ability to activate RTK multimerization and/or to activate the RTK not limited to the phosphorylation activity. In one or more embodiments, the positive evaluation of the effect on the molecular function of the RTK includes the evaluation that the target substance exhibits an activation effect on the RTK or can become an agonist of the RTK.

In one or more embodiments, the threshold may be determined based on the value (e.g., KLD value) obtained by quantifying the distribution of the degree of RTK multimerization obtained under the control condition (without contact of the target substance). In one or more embodiments, the threshold may also be determined based on the value (e.g., KLD value) obtained under a positive control (e.g., a substance known as the ligand for the RTK to be evaluated is added).

[Method for screening]

In still another aspect, the present disclosure relates to a method for screening a candidate inhibitor (antagonist candidate) or activator (agonist candidate) targeting the RTK, including evaluating the effect of the target substance on the molecular function of the RTK by the method for evaluating the RTK activity of the present disclosure described above. In still another aspect, the present disclosure relates to a method for screening a candidate substance that inhibits the RTK's molecular function and/or activity (antagonist candidate) or a candidate substance that activates the RTK (agonist candidate). The method includes quantifying the RTK's molecular function and/or activity by the target substance, and/or determining a 50% effective concentration ($EC_{50}$) of the target substance or a ligand on the RTK by a method for determining the quantified value of the RTK's molecular function and/or activity of the present disclosure or by a method for determining the $EC_{50}$ of the target substance or the ligand on the RTK's molecular function and/or activity of the present disclosure.

[Program]

In still another aspect, the present disclosure relates to a program that causes a computer to execute the method for evaluating the RTK activity of the present disclosure and/or a program that makes the screening method of the present disclosure to be executed, and to a program product in which the program is recorded. In one or more embodiments, the computer in the present disclosure may be realized using one or more processors such as a central processing unit (CPU).

The present disclosure may relate to one or more of the following embodiments:

[1] a method for evaluating an RTK activity, the method including:

a step (A1) for determining diffusion dynamics A1 of the RTK by contacting a target substance with a cell that expresses a labeled RTK on a cell membrane;
a step (B1) for determining diffusion dynamics B1 of the RTK by contacting the target substance and a ligand that activates the RTK with a cell that expresses the labeled RTK on a cell membrane;
a step (C1) for calculating a ratio of the diffusion dynamics A1 of the RTK obtained at the step (A1) and the diffusion dynamics B1 of the RTK obtained at the step (B1) (diffusion dynamics B1 / diffusion dynamics A1); and
a step (D1) for evaluating an effect of the target substance on the RTK activity by comparing the ratio of the diffusion dynamics obtained at the step (C1) with a threshold, in which
at the step (D1), the ratio of the diffusion dynamics obtained at the step (C1) being equal to or greater than the threshold can indicate that the target substance has an effect on a molecular function of the RTK;

[2] the method according to [1], further including:

a step (E1) for determining diffusion dynamics E1 of the RTK without contacting the target substance and the ligand that activates the RTK with a cell that expresses the RTK on a cell membrane;
a step (F1) for determining diffusion dynamics F1 of the RTK by contacting the ligand that activates the RTK, without contacting the target substance, with a cell that expresses the RTK on a cell membrane; and
a step (G1) for calculating a ratio of the diffusion dynamics E1 of the RTK obtained at the step (E1) and the diffusion dynamics F1 of the RTK obtained at the step (F1) (diffusion dynamics F1 / diffusion dynamics E1), in which
the comparison of the ratio of the diffusion dynamics (diffusion dynamics B1 / diffusion dynamics A1) with the threshold at the step (D1) includes calculation of the difference between the ratios of the diffusion dynamics obtained at the step (C1) (diffusion dynamics B1 / diffusion dynamics A1) and the ratio of diffusion dynamics obtained at the step (G1) (diffusion dynamics F1 / diffusion dynamics E1) ([diffusion dynamics B1 / diffusion dynamics A1] - [diffusion dynamics F1 / diffusion dynamics E1]), and comparison of the difference of the obtained ratios of the diffusion dynamics ([diffusion dynamics B1 / diffusion dynamics A1] - [diffusion dynamics F1 / diffusion dynamics E1]) with a threshold, and

the difference between the ratios of the diffusion dynamics ([diffusion dynamics B1 / diffusion dynamics A1] - [diffusion dynamics F1 / diffusion dynamics E1]) being equal to or greater than the threshold can be evaluated to indicate that the target substance has an effect on the molecular function of the RTK;

[3] the method according to [1], further including:

a step (E1) for determining diffusion dynamics E1 of the RTK without contacting the target substance and the ligand that activates the RTK with a cell that expresses the RTK on a cell membrane;
a step (F1) for determining diffusion dynamics F1 of the RTK by contacting the ligand that activates the RTK, without contacting the target substance, with a cell that expresses the RTK on a cell membrane; and
a step (G1) for calculating a ratio of the diffusion dynamics E1 of the RTK obtained at the step (E1) and the diffusion dynamics F1 of the RTK obtained at the step (F1) (diffusion dynamics F1 / diffusion dynamics E1 ), in which the threshold is determined based on the ratio of the diffusion dynamics obtained at the step (G1);

[4] a method for evaluating an RTK activity, the method including:

a step (A2) for determining diffusion dynamics A2 of the RTK without contacting a target substance with a cell that expresses a labeled RTK on a cell membrane;
a step (B2) for determining diffusion dynamics B2 of the RTK by contacting the target substance with a cell that expresses the labeled RTK on a cell membrane;
a step (C2) for calculating a ratio of the diffusion dynamics A2 of the RTK obtained at the step (A2) and the diffusion dynamics B2 of the RTK obtained at the step (B2) (diffusion dynamics B2 / diffusion dynamics A2) or a difference between the diffusion dynamics B2 and the diffusion dynamics A2 (diffusion dynamics B2 - diffusion dynamics A2);
a step (E2) for determining diffusion dynamics E2 of the RTK without contacting the target substance with a cell that expresses the labeled RTK on a cell membrane;
a step (F2) for determining diffusion dynamics F2 of the RTK by contacting a solution not containing the target substance with the cell at the step (E2);
a step (G2) for calculating a ratio of the diffusion dynamics E2 of the RTK obtained at the step (E2) and the diffusion dynamics F2 of the RTK obtained at the step (F2) (diffusion dynamics F2 / diffusion dynamics E2), or a difference between the diffusion dynamics E2 and the diffusion dynamics F2 (diffusion dynamics F2 - diffusion dynamics E2); and
a step (D2) for evaluating the RTK activity of the target substance by comparing an absolute value of the difference between the ratio of the diffusion dynamics obtained at the step (C2) (diffusion dynamics B2 / diffusion dynamics A2) and the ratio of the diffusion dynamics obtained at the step (G2) (diffusion dynamics F2 / diffusion dynamics E2) ([diffusion dynamics B2 / diffusion dynamics A2] - [diffusion dynamics F2 / diffusion dynamics E2]), or an absolute value of the difference between the diffusion dynamics difference obtained at the step (C2) ([diffusion dynamics B2 - diffusion dynamics A2]) and the diffusion dynamics difference obtained at the step (G2) ([diffusion dynamics F2 - diffusion dynamics E2]) ([diffusion dynamics B2 - diffusion dynamics A2] - [diffusion dynamics F2 - diffusion dynamics E2]) with a threshold, in which
at the step (D2), the ratio of the diffusion dynamics or the difference of the diffusion dynamics obtained at the step (G2) being equal to or greater than the threshold can indicate that the target substance has an effect on the molecular function of the RTK not limited to the phosphorylation activity;

[5] a method for determining a quantified value of the RTK's molecular function and/or activity, and/or a 50% effective concentration ($EC_{50}$) of a target substance or a ligand on the RTK's molecular function and/or activity, the method including:

a step (A3) for determining diffusion dynamics A3 of the RTK without contacting a target substance and a ligand that activates the RTK with a cell that expresses a labeled RTK on a cell membrane;
a step (B3) for determining diffusion dynamics B3 of the RTK by contacting the target substance or the ligand that activates the RTK with a cell that expresses the labeled RTK on a cell membrane;
a step (C3) for calculating a ratio of the diffusion dynamics A3 of the RTK obtained at the step (A3) and the diffusion dynamics B3 of the RTK obtained at the step (B3), (diffusion dynamics B3 / diffusion dynamics A3); and
a step (D3) for determining a quantified value of an effect on the RTK's molecular function and/or activity caused by the target substance or the ligand, and/or a 50% effective concentration ($EC_{50}$) of the target substance or the ligand on the RTK's molecular function and/or activity, by using the ratio of the diffusion dynamics (diffusion dynamics B3 / diffusion dynamics A3) obtained at the step (C3) ;

[6] the method according to any of [1] to [5], in which the diffusion dynamics is determined as a mean square displacement or as a diffusion coefficient;

[7] the method according to any of [1] to [6], in which the label is a fluorescent label, and the diffusion dynamics is measured using fluorescent single-molecule imaging;

[8] a method for evaluating an RTK activity, the method including:

a step (A4) for contacting a target substance with a cell that expresses a labeled RTK on a cell membrane;
a step (B4) for contacting the target substance and a ligand that activates the RTK with a cell that expresses the labeled RTK on a cell membrane;
a step (C4) for quantifying distributions of the degree of RTK multimerization at the step (A4) and at the step (B4); and
a step (D4) for evaluating the RTK activity of the target substance by comparing the value obtained at the step (C4) with a threshold, in which
the difference between the value obtained at the step (C4) and the threshold being equal to or greater than a predetermined value at the step (D4) can indicate that the target substance has an effect on the molecular function of the RTK;

[9] the method according to [8], in which the distribution at the step (C4) is determined as a distribution of luminance of the label or a diffusion coefficient distribution of the label;

[10] the method of RTK according to [8] or [9], in which the quantification at the step (C4) is performed by KL divergence;

[11] a method for evaluating an RTK activity, the method including:

a step (A5) for contacting a target substance with a cell that expresses a labeled RTK on a cell membrane;
a step (B5) for quantifying a distribution of a degree of RTK multimerization before and after the contact of the target substance; and
a step (C5) for evaluating the target substance on the RTK's molecular function and/or activity by comparing the value obtained at the step (B5) with a threshold, in which
the difference between the value obtained at the step (B5) and the threshold being equal to or greater than a predetermined value at the step (C5) can indicate that the target substance has an effect on the RTK's molecular function and/or activity;

[12] the method according to [11], in which the distribution at the step (B5) is determined as a distribution of luminance of the label or a diffusion coefficient distribution of the label;

[13] the method according to [11] or [12], in which the quantification at the step (B5) is performed by KL divergence;

[14] the method according to any of [8] to [13], in which the label is a fluorescent label, and the distribution is measured using fluorescent single-molecule imaging;

[15] the method according to any of [1] to [14], in which the RTK is at least one selected from the group consisting of EGFR, ERBB2, ERBB3, and ERBB4;

[16] a method for screening an inhibitor candidate or an activator candidate targeting an RTK, the method including the method according to any of [1] to [15];

[17] a method for screening an inhibitor candidate or an activator candidate targeting the RTK, the method including: evaluating an effect of the target substance on the RTK's molecular function by any of the methods according to [1] to [4] and [6] to [15], and/or quantifying the target substance on the RTK's molecular function and/or activity, and/or determining the $EC_{50}$ of the target substance or the ligand on the RTK by the methods according to [5] to [7], [14], and [15]; and

[18] the method according to [16] or [17], in which the RTK is at least one selected from the group consisting of EGFR, ERBB2, ERBB3, and ERBB4.

Brief Description of Drawings

FIG. 1 is the probability distribution diagram used to explain the KL divergence calculation method.
FIG. 2 is one example of the measurement result of the EGFR phosphorylation using the Western blotting method in Experimental Example 1.

FIGS. 3A and 3B are examples of the images obtained by single-molecule imaging in Experimental Example 2; FIG. 3A is the image of the ligand (EGF) unstimulated CHO cell and FIG. 3B is the image of the ligand (EGF 60 nM) stimulated CHO cell.

FIGS. 4A and 4B are graphs obtained as a result of the analysis in Experimental Example 3, illustrating the relationship between the EGF concentration and the phosphorylation of EGFR (fold, left vertical axis) and the diffusion dynamics of EGFR (right vertical axis). The phosphorylation in FIG. 4A is the result of using pY1173, and the phosphorylation in FIG. 4B is the result of using pY1068.

FIG. 5 is one example of the results of drug evaluation using the diffusion dynamics ratio (MSD ratio) in Experimental Example 5.

FIG. 6 is a graph illustrating one example of the results of the drug screening of the FDA-approved drug library using the method of the present disclosure.

FIG. 7 is a graph illustrating one example of the results of the drug screening of the FDA-approved drug library using the method of the present disclosure.

FIGS. 8A and 8B are graphs obtained as a result of the analysis in Experimental Example 6, showing the relationship between the drug (Sapitinib or Afaitinib) concentration and the diffusion dynamics ($MSD\delta_{t=500\,ms}$ ratio) of the receptor (ErbB3 or ErbB4): FIG. 8A shows the result of Sapitinib and FIG. 8B shows the result of Afaitinib. The $IC_{50}$ in FIGS. 8A and 8B is the 50% inhibitory concentration obtained from the $MSD\delta_{t=500\,ms}$ ratio.

[Examples]

Hereinafter, the present disclosure will be described in more detail by Examples and Experimental Examples, but they are illustrative only and the present disclosure is not limited to these Examples. All references cited in the present disclosure are incorporated as part of the present disclosure.

[Experimental Methods]

1) Single-molecule imaging

Using the Automated in-cell Single-Molecule Imaging System (AiSIS) developed by the inventors of the present invention, single-molecule imaging of the EGFR was performed in the CHO cell by GFP fluorescence. Single-molecule imaging was performed using the following procedure.

(1) The CHO cell that expresses the EGFR-GFP having the GFP fused to the EGFR was prepared.
(2) The 96-well plate having the CHO cell placed therein is placed in the port of the AiSIS, and then the automatic imaging is started.
(3) The 96-well plate is automatically moved to the microscope stage and set on the objective lens.
(4) Coarse and fine autofocusing is performed to focus on the coverslip surface.
(5) Perform cell search for 300 regions to determine the 10 regions indicating the highest evaluation value (E).
(6) Image acquisition is performed for five regions having evaluation values in odd-numbered ranks.
(7) Add a chemical as necessary and perform the image acquisition for the five regions having evaluation values in even-numbered ranks.
(8) Move the next well into the prescribed position and repeat the operations (4) through (7) for a predetermined period of time. The snapshots of the surface reflection interference contrast (SRIC), which are performed to know the exact area of the cell, are taken in all areas before the fluorescent images are acquired with 100 frames.

2) Analysis of images of single-molecule imaging

After acquiring the images by single-molecule imaging in 1) above, the single-molecule tracking (SMT) and additional analysis are performed using the SMT software that is developed by the inventors of the present invention.

In the analysis of the images of single-molecule imaging, first the position of the center of gravity of each fluorescent spot is calculated over the detected frames. By obtaining the trajectory from the spatio-temporal information of these bright spots, it is possible to know the localization and dynamic properties of the molecule. Because the luminance of the bright spot reflects the number of the fluorescent molecules contained, it is possible, for example, to follow the process of formation and decomposition of the multimer.

That is, first, the fluorescent spot is detected within the cellular region determined by the deep learning of the SRIC image. The cross-correlation between all pixels of the obtained image and the intensity profile ($I_{i,j}$) following a Gaussian distribution is calculated using the following formula.

[Math. 5]

$$y_{i,j} = \frac{\sum_{I=-R}^{R}\sum_{J=-R}^{R}(l_{I,J} - l_{avg})(x_{i+I,\,j+J} - x_{avg})}{\sqrt{\sum_{I=-R}^{R}\sum_{J=-R}^{R}(l_{I,J} - l_{avg})^2}\sqrt{\sum_{I=-R}^{R}\sum_{J=-R}^{R}(x_{i+I,\,j+J} - x_{avg})^2}}$$

$$l_{i,j} = \frac{1}{\sqrt{2\pi}\sigma}\exp\left(-\frac{i^2 + j^2}{2\sigma^2}\right)$$

$$l_{avg} = \frac{1}{(2R+1)^2}\sum_{I=1}^{R}\sum_{J=1}^{R}l_{I,J}$$

$$x_{avg} = \frac{1}{(2R+1)^2}\sum_{I=1}^{R}\sum_{J=1}^{R}x_{i+I,\,j+J}$$

Here, $x_{i,j}$ and $y_{i,j}$ each represent the pixel intensities at $(i,j)$ in the cross-correlation image obtained. R represents the length of the side of a square ROI (5 pixels). In $l_{i,j}$, $(i,j) \in$ [-5 to 5], and σ represents the standard deviation set at two pixels.

Then, binarization is performed on $y_{i,j}$ with the threshold of 0.25. The value of pixels larger than the threshold is labeled as the fluorescent spot. The center of the spot is then determined by fitting the intensity of the ROI in the raw image using the following formula.

[Math. 6]

$$I(x, y; I_0, x_g, y_g, \sigma_A, a, b, I_{back}) = I_0\exp\left(\frac{(x - x_g)^2 + (y - y_g)}{2\sigma_A^2}\right) + a(x - x_g) + b(y - y_g) + I_{back}$$

Here, $(x_g, y_g)$ is the centroid of the Gaussian function of the peak intensity $I_0$ and variance $\sigma_{A2}$.

The Gaussian function is located on the inclined plane (a and b) above the offset intensity $I_{back}$. The spots with $\{\sigma_A\}<1.5$ or $\{\sigma_A\}>2.5$ and centers located outside the cell area are discarded. The obtained spot information consists of the time, the position, and the fluorescence intensity. The spot trajectory is created by connecting the centers of consecutive frames with the interval of less than 6 pixels.

The mean square displacement (MSD) of each trajectory in the time interval nδt is calculated using the following formula.

[Math. 7]

$$\text{MSD}(n \cdot \delta t) = \frac{1}{N-1-n}\sum_{j=1}^{N-1-n}\{[x(j \cdot \delta t + n \cdot \delta t) - x(j \cdot \delta t)]^2 + [y(j \cdot \delta t + n \cdot \delta t) - y(j \cdot \delta t)]^2\}$$

(1)

Here, N represents the total frame length of the trajectory, n represents the frame length, δt represents the frame rate (time interval between frames), j represents the initial frame, and **(x,** y) represents the molecular position.

3) Western blotting

Protein phosphorylation was quantified by Western blotting using antibodies against pEGFR (pY1173: #4407 and pY1068: #3777; both obtained from Cell Signaling Technology (CST)). Antibody binding was detected by the luminescence using the HRP-conjugated anti-IgG antibody diluted 1:2000 (rabbit: #7074, mouse: #7076; both were obtained from CST) and the ECL prime reagent (GE Healthcare) as a secondary antibody. The luminescence intensity was measured using the ImageJ software (NIH). The rectangular regions of interest were established in the signal (band) and background (far enough from the signal) regions. The difference between the average intensities of the two regions was defined as the band intensity. In the time course analysis, the fold-change of the phosphorylation level at 0 min was calculated. The band intensities at each time point obtained under all conditions were normalized against the band intensity at 1 min time point for the control cell measured in the same experiment. The intensity at minute 0 is very weak and, coupled this with the relatively high noise, this is not suitable as the normalization coefficient. The 0-minute normalized values for all conditions were then averaged; and this was used as the denominator for the values at each time point. For the dose-response analysis, under all conditions the band intensities obtained at each EGF concentration were normalized against the band intensity at 300 nM EGF in the control cell measured in the same experiment. The dose-response curve was fitted using the following Hill equation.

[Math. 8]

$$phosphorylation = bottom + \frac{top - bottom}{1 + \left(\frac{EC_{50}}{[EGF]}\right)^n}$$

In the Hill equation, n, top, and bottom are the Hill coefficient, upper and lower limit fitting parameters, respectively, and [EGF] is the concentration of EGF.

4) Calculation of KL divergence (KLD value)

The KLD value is calculated as follows.

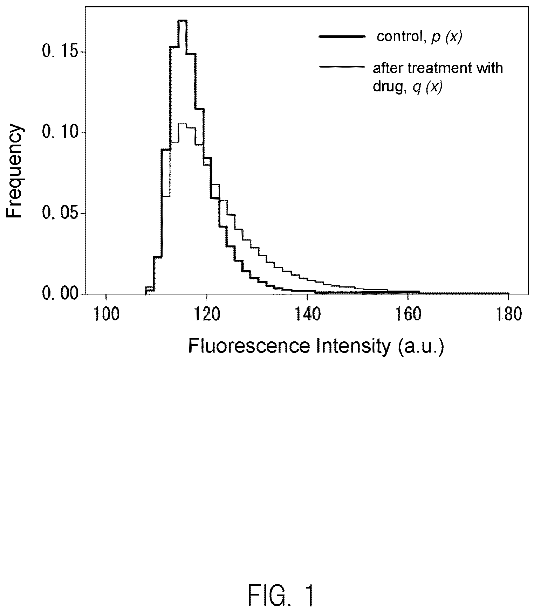

(1) A histogram of probability distribution is created from the frequency distribution of the fluorescence intensity obtained by single-molecule imaging for each of the control condition (addition of buffer only, i.e., before treatment with the ligand or with the target substance) and after the treatment with the ligand or with the target substance (FIG. 1). (2) Calculate the A value using the frequency for each histogram bin interval according to the following formula.

[Math. 9]

$$A = p(x)\ln\frac{p(x)}{q(x)}$$

p(x): Frequency in the interval x in the probability distribution of the control (before treatment with the ligand or with the target substance)
q(x): Frequency in the interval x in the probability distribution after treatment with the ligand or with the target substance

(3) Calculate the KLD value (KL(p∥q)) by summing up A in all the bin intervals according to the following formula.

[Math. 10]

$$KL(p \parallel q) = \int_{-\infty}^{\infty} p(x)\ln\frac{p(x)}{q(x)}dx$$

[Experimental Example 1] Measurement of phosphorylation of EGFR using Western blotting method

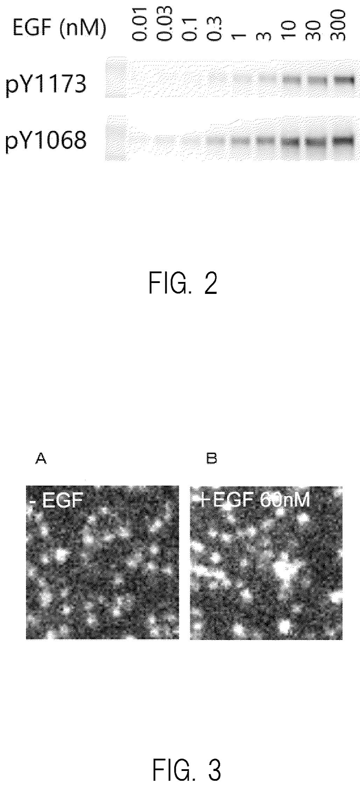

A dose-response curve was obtained by measuring the phosphorylation of the EGFR according to the method described above for the Western blotting method. The concentrations of EGF were 0.01, 0.03, 0.1, 0.3, 1, 3, 10, 30, and 300 nM. One example of the results of the Western blot analysis is shown in FIG. 2. The $EC_{50}$ of pY1173 was 1.9 nM and $EC_{50}$ of pY1068 was 1.5 nM.

[Experimental Example 2] Single-molecule imaging

The large-scale single-molecule analysis of the EGFR-mediated signaling was performed by single-molecule imaging using the AiSIS described above.

After stimulating the CHO cells having the EGFR-GFP expressed with 60 nM EGF, the images with the time lapse of 10 minute were acquired with 15 intervals from a total of more than several hundred cells. One example of the resulting image is shown in FIG. 3. FIG. 3 is the images obtained by single-molecule imaging, where FIG. 3A is the cell image without the EGF stimulation and FIG. 3B is the cell image after stimulation with a 60 nM EGF.

As can be seen in FIG. 3, the images obtained by single-molecule imaging using the AiSIS showed that the diffusion motion of the EGFR was reduced and that the EGFR was multimerized in the cells after stimulation with a 60 nM EGF (FIG. 3B) as compared to the cells without the EGF stimulation (FIG. 3A).

[Experimental Example 3] Image analysis obtained by single-molecule imaging

First, the CHO cells having the EGFR-GFP expressed were placed in the multiplate (96 wells), and single-molecule imaging was performed in the absence of the EGF, as described above. The images by single-molecule imaging were acquired with 15 intervals from a total of more than several hundred cells. The acquired single-molecule images were automatically recorded, and the positional information and fluorescence intensity data of individual molecules were obtained from the resulting single-molecule-resolution cellular moving images.

Then, a buffer solution with different concentration of EGF or a buffer solution without EGF was added to the above wells, and then single-molecule imaging was performed in the same way as above to obtain the image with the time lapse of 10 minutes after the addition of the buffer solution. The EGF concentrations in the buffer solution were 0.01, 0.03, 0.1, 0.3, 1, 3, 10, 30, and 300 nM.

Based on the positional information of individual molecules obtained by the above single-molecule imaging, the $MSD\delta_{t=500\,ms}$ (MSD value) was calculated for each well (cell) with 16 frames ($\delta t = 500$ ms) using the above formula (1). The obtained MSD values were then averaged for each well with the same EGF concentration. The "MSD value ($MSD\delta_{t=500\,ms}$)" in Experimental Examples below refers to the well-averaged MSD value ($MSD\delta_{t=500\,ms}$).

The obtained MSD values ($MSD\delta_{t=500\,ms}$) were then used to calculate the ratio of the MSD values before and after the EGF addition ($MSD\delta_{t=500\,ms}$ ratio) from the following formula (2). The $MSD\delta_{t=500\,ms}$ ratios were obtained for all the EGF concentrations (0.01, 0.03, 0.1, 0.3, 1, 3, 10, 30, and 300 nM) .

$$[MSD\delta_{t=500\,ms}\ ratio] = MSD_{after\ EGF\ addition}\ /\ MSD_{before\ EGF\ addition}$$

$$(2)$$

As a result, the MSD ratio in the EGF-added (EGF-stimulated) wells decreased dramatically compared to that before the EGF stimulation, whereas the MSD ratio in the EGF-unadded (EGF-unstimulated) wells (mock) did not change before and after the addition of the buffer solution. The statistical tests showed the significant change between the MSD ratio before and after, suggesting that a receptor activation (phosphorylation) may be recognized as the behavioral change. It was also suggested that there is an EGF concentration dependence in the MSD value ratio.

The half-maximum effective concentration ($EC_{50}$) of the EGF for the behavior of the EGFR was calculated from the following formula (3). The resulting $EC_{50}$ was 1.5 nM.

[Math. 11]

$$\mathbf{MSD} = \mathbf{MSD_{max}} - \frac{(\mathbf{MSD_{max}} - \mathbf{MSD_{min}})[L]}{[L] + \mathbf{EC_{50}}} \qquad (3)$$

MSD: well-averaged $MSD\delta_{t=500\,ms}$ ratio
$MSD_{max}$: Maximum value of the averaged $MSD\delta_{t=500\,ms}$ ratio
$MSD_{min}$: Minimum value of the averaged $MSD\delta_{t=500\,ms}$ ratio
[L]: concentration of the EGF (ligand) in the well used for the MSD value.

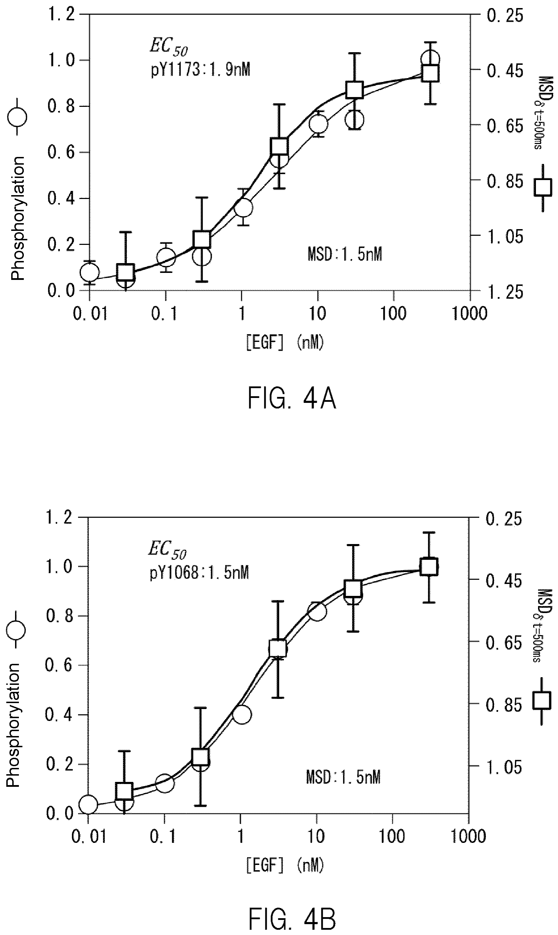

Next, the correlation between the phosphorylation of the receptor and the diffusion dynamics of the receptor was further analyzed based on the results of the Western blot analysis in Experimental Example 2 and the MSD value ($MSD\delta_{t=500\,ms}$). The results are shown in FIGS. 4A and 4B. FIGS. 4A and 4B are graphs showing the relationship between the EGF concentration and the phosphorylation of the receptor obtained by the Western blotting (fold, left vertical axis) and the diffusion dynamics of the receptor obtained by single-molecule imaging (right vertical axis, $MSD\delta_{t=500\,ms}$ ratio), where the phosphorylation in FIG. 4A is the result using pY1173 in Western blotting, and the phosphorylation in FIG. 4B is the result using pY1068 in Western blotting. As can be seen in the graphs in FIGS. 4A and 4B, the activation (autophosphorylation) of the receptor (EGFR) is proportional to the decrease in the diffusion motility of the receptor. Therefore, it was confirmed that the autophosphorylation of the receptor (EGFR) can be quantitatively estimated and that the signaling can be inferred, by measuring the diffusion motion of the RTK receptor (EGFR) and calculating the ratio of the MSD values.

These results indicate that the diffusion motion (ratio of the MSD values) of the receptor (EGFR) obtained by single-molecule imaging can be used to quantitatively evaluate the signaling activity by the EGFR autophosphorylation.

[Experimental Example 4] Single-molecule imaging using inhibitor

The same procedure as in Experimental Example 3 above was used, except that the cell treated with gefitinib (10,000 nM), which is a drug that inhibits EGFR signaling, was used. As a result, in the gefitinib-treated cell, the stimulation with the EGF did not lower the ratio of the MSD values as recognized in Experimental Example 3 above. With this, it became apparent that it is possible to evaluate the substance (compound) that has the effect on the RTK's molecular function, such as a phosphorylation inhibitor, by the change in the diffusion dynamics of the EGFR (ratio of the MSD values).

[Experimental Example 5] Drug evaluation using the diffusion dynamics (MSD ratio)

As can be seen in Experimental Example 3 and in FIGS. 4A and 4B, it was confirmed that the MSD value, which represents the molecular movement, has a linear relationship with the amount of the EGFR phosphorylation. For this reason, the inventors of the present invention attempted to design a drug evaluation using the MSD value (diffusion dynamics of the EGFR). During the course of the study, the inventors of the present invention found that the MSD value fluctuates not only with the EGF (ligand) but also with stimulation by the candidate compound (target substance), and that simply evaluating the absolute MSD value is not sufficient to accurately evaluate the effect of the candidate compound on the EGFR activation by the EGF. Therefore, the ratio of MSD values before and after the addition of the EGF (ligand) ($MSD\delta_{t=500\,ms}$ ratio) was calculated, and the drug evaluation experiment was performed using the obtained $MSD\delta t_{=500\,ms}$ ratio.

Single-molecule imaging was performed in the same way as in Experimental Example 3, except that the cell treated with the gefitinib (EGFR inhibitor) was used as the CHO cell that expresses the EGFR-GFP and that the EGF concentration in the buffer solution was made 60 nM. First, the CHO cells treated with the gefitinib (10,000 nM) were placed in the multiplate (96 wells) and single-molecule imaging was similarly performed in the absence of the EGF. Then, the EGF-containing buffer solution was added to each well, and single-molecule imaging was performed in the presence of the EGF. The analysis of the images obtained by single-molecule imaging was performed to calculate the $MSD\delta_{t=500\,ms}$ before and after the EGF addition for each well, and the ratio of the MSD values before and after the EGF addition ($MSD\delta_{t=500\,ms}$ ratio) was calculated using the obtained $MSD\delta_{t=500\,ms}$ from the following formula (4).

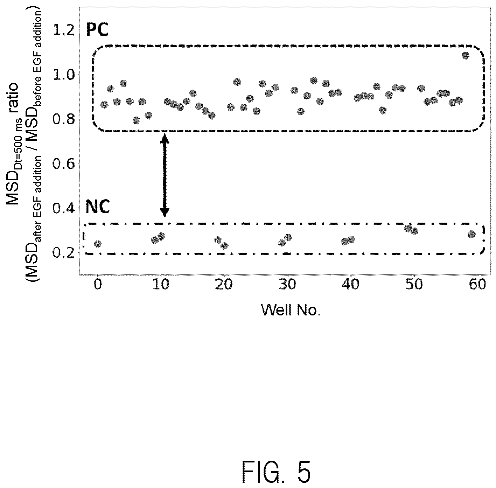

Single-molecule imaging was performed in the same way as above, except that the gefitinib-untreated CHO cell was used instead of the gefitinib-treated CHO cell as the control, and then the $MSD\delta_{t=500\,ms}$ ratio was calculated. The results are shown in FIG. 5.

$$[MSD\delta_{t=500\,ms}\ ratio] = MSD_{after\ EGF\ addition}\ /\ MSD_{before\ EGF\ addition}$$

(4)

In FIG. 5, the EGFR inhibitor-treated group (PC) is surrounded by a dashed line and the EGFR inhibitor-untreated group (NC) is surrounded by a single dotted line. As can be seen in FIG. 5, by comparing the EGFR movement using the $MSD\delta_{t=500\,ms}$ ratio, it was possible to distinguish very clearly between the EGFR inhibitor-treated (PC) and the untreated (NC) groups. In addition, the Z'-factor, which is the indicator of the accuracy, was very high with the value of 0.65.

The reasons for this high accuracy may be inferred as follows. The MSD value changes not only with stimulation by the EGF (ligand) as described above, but also with stimulation by the treating compound. Therefore, by calculating the ratio of the MSD values ($MSD\delta_{t=500\,ms}$ ratio) before and after the addition of the EGF (ligand), the ratio of the MSD values obtained will be the value that cancels out the change in the molecular motion associated with the treating compound, resulting in a more accurate reading of only the molecular motion related to the activation that may occur by EGF (ligand). Therefore, it is believed that the accurate evaluation can be made on the effect of the treating compound (EGFR inhibitor) on the activation of the EGFR caused by binding of the EGF (ligand) to the EGFR. In addition, the MSD value calculated based on the positional information obtained by single-molecule imaging may lead to a quantitative estimation of the degree of activation based on the movement of the EGFR molecule, rather than directly evaluating the EGFR phosphorylation, as is the case with the Western blotting. These findings suggest that the ratio of the MSD values may be used to realize a novel, quantifiable drug evaluation method that allows for compound/drug exploration.

[Drug screening of FDA-approved drug library using MSD$\delta_{t=500}$ ms ratio]

Based on the above findings, single-molecule imaging was used to screen the drugs using the MSD$\delta_{t=500\,ms}$ ratio on the FDA-approved drug library (1134 drugs), including known EGFR inhibitors and kinase inhibitors.

<Evaluation method>

Using the AiSIS as described above, single-molecule imaging was performed in the same way as in Experimental Example 3, except that the CHO cell having the EGFR-GFP expressed and a candidate compound were placed in the wells with the EGF concentration in the buffer solution being set at 60 nM.

That is, first, the CHO cell that expresses the EGFR-GFP and the candidate compound were placed in a multiplate (96 wells), and then single-molecule imaging was performed in the absence of the EGF. Then, the EGF-containing buffer solution was added to each well to perform single-molecule imaging in the presence of the EGF. The analysis of the images obtained by single-molecule imaging was performed to calculate the MSD$\delta_{t=500\,ms}$ before and after the EGF addition for each candidate compound, and the MSD$\delta_{t=500\,ms}$ ratio before and after the EGF addition for each compound.

[MSD$\delta_{t=500\,ms}$ ratio of candidate compound] = MSD$_{after\,EGF}$ addition / MSD$_{before}$ EGF addition

Single-molecule imaging was also performed for the control in order to determine the threshold. Single-molecule imaging for the control was performed in the same way as above, except that the candidate compound was not placed in the wells. The analysis of the images obtained by single-molecule imaging was performed; so, the MSD$\delta_{t=500\,ms}$ ratio (control value) before and after the EGF addition was calculated, and the average and standard deviation thereof were calculated.

[MSD$\delta_{t=500\,ms}$ ratio of control] = MSD$_{after\,EGF}$ addition / MSD$_{before}$ EGF addition

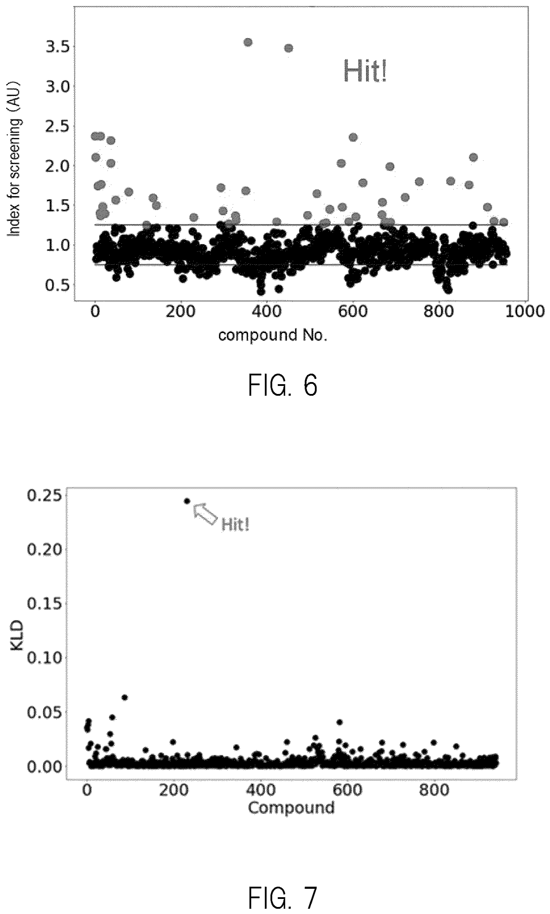

The value more than 3 times the standard deviation away from the average of the control value was set as the threshold ([threshold] = average value of MSD$\delta_{t=500\,ms}$ ratio of control + 3 × standard deviation). Among the candidate compounds, the compound having the MSD$\delta_{t=500\,ms}$ ratio equal to or greater than the threshold, that is, the compound showing the point where the difference between [MSD$\delta_{t=500\,ms}$ ratio of the candidate compound] and [MSD$\delta_{t=500\,ms}$ ratio of the control] was away by more than three times the standard deviation was designated as the hit compound. As a result, 53 compounds out of 1134 candidate compounds hit, as can be seen in FIG. 6. In FIG. 6, the "index for screening" is the value obtained by normalizing the MSD$\delta_{t=500\,ms}$ ratio of the candidate compound by the average value of the MSD$\delta_{t=500\,ms}$ ratio of the control.

Single-molecule imaging was performed again for the hit candidate compounds under five conditions of different orders of the EGF concentration (EGF concentration: 1, 10, 100, 1,000, and 10,000 nM). The MSD$\delta_{t=500\,ms}$ ratio was measured for 5 EGF concentration conditions of different orders to obtain the concentration dependence of the effect of each candidate compound (e.g., phosphorylation inhibition effect). Compounds that are within a significance level of 5% and increased in a concentration-dependent manner were selected, from which the compounds that are difficult to be measured due to coloration or cause the cell damage were excluded. As a result, 12 compounds were selected from the above 53 compounds.

Of the 12 compounds obtained from the screening, 7 compounds (Gefitinib, Afatinib, Erlotinib, Lapatinib, Vandetanib, Dasatinib, and Ibrutinib) were drugs known to inhibit the EGFR kinase activity and precisely included the EGFR kinase inhibitors in the FDA-approved drug library used. This demonstrated the validity of the method for evaluating an activity of the present invention.

On the other hand, with regard to the remaining 5 compounds (Eltrombopag, Broxyquinoline, Sorafenib, Glafenine, and Nilotinib), there have been no reports about the effect on the EGFR. In these compounds, the MSD$\delta_{t=500\,ms}$ ratio was significantly different from the control because the addition of them significantly changed the MSD$\delta_{t=500\,ms}$ (in an EGF-independent manner). In other words, it was indicated that these compounds have the effect on molecular function other than the activation (phosphorylation) of the EGFR, which is inhibited by a kinase inhibitor. Therefore, the effect of these compounds on the subcellular localization of the EGFR was examined by the EGFR immunostaining, and then, it was found that the EGFR was incorporated into the cell by these compounds. The EGFR is known to be incorporated into the cell by bonding to the EGF. On the other hand, the EGF was not necessary for incorporation of the EGFR into the cell by treatment with these compounds. Thus, it became apparent that the action of the five compounds screened in this study changed the subcellular localization of the EGFR.

These results indicate that the compound/drug screening is feasible by using the $MSD\delta_{t=500\,ms}$ ratio, one of the quantitative indicators to characterize the single-molecule dynamics of the EGFR, and that a new compound/drug that could not be detected by existing methods can be explored.

By the way, in the above 53 compounds, other than the above 12 compounds, it has been confirmed that OSI-420 (a derivative of Erlotinib) and Lapatinib ditosylate (a derivative of Lapatinib), which are drugs known to inhibit the kinase activity of the EGFR, are included, and that curcumin, which is a food additive known to inhibit the tyrosine kinases including EGFR, is also included.

From further analysis of the data, it was confirmed that in the 53 compounds listed above, Ponatinib, a pantyrosine kinase inhibitor, and Daunorubicin (an anthracycline antibiotic), the compound whose effect on the EGFR has not been reported, are included.

[Drug screening of FDA-approved drug library using KL divergence]

By performing single-molecule imaging, the fluorescence intensity can be obtained along with the positional information of individual molecules used to calculate the MSD value. The inventors of the present invention obtained the information that distribution of the fluorescence intensity may be used as a characteristic value of the multimer formation of the receptor (EGFR), and that by using the KL divergence for the method for quantitatively evaluating the frequency distribution of this fluorescence intensity, a new drug evaluation method that enables to explore a compound/drug that has been difficult to be detected by existing methods can be realized.

Based on this information, single-molecule imaging was used to screen the drugs using the KL divergence for the FDA-approved drug library (1134 drugs), including known EGFR inhibitors and kinase inhibitors.

<Evaluation method>

Single-molecule imaging was performed in the same way as Experimental Example 3 using the AiSIS as described above, except that the buffer solution containing the candidate compound was used instead of the EGF, and the fluorescence intensity obtained was used to create a histogram of probability distribution from the frequency distribution of the fluorescence intensity to calculate the KLD value. The concentration of the candidate compound in the buffer solution was set to 10,000 nM.

The KLD value was calculated for each well (cell) as described in the section "4) Calculation of KL divergence (KLD value)". In the formula (5) below, q represents the probability distribution created using the fluorescence intensity 1.5 minutes after the addition of the candidate compound in the wells in which the CHO cells were placed, and p represents the probability distribution created using the fluorescence intensity before the addition of the candidate compound.

[Math. 12]

$$KL(p \parallel q) = \int_{-\infty}^{\infty} p(x)\ln\frac{p(x)}{q(x)} dx \qquad (5)$$

For the threshold KLD value, the fluorescence intensity before and after the addition of the EGF-containing buffer solution obtained by single-molecule imaging was used to calculate the KLD value in the same way as above, and the average value and standard deviation thereof were calculated. The control is the KLD value calculated in the same way as above using the fluorescence intensity before and after the addition of only the solvent used to dissolve the compound.

[Threshold] = KLD value of control (average) + significant difference

The compound that showed a point where the KLD value of the candidate compound was significantly away from the average of the threshold KLD values was designated as the hit compound.

As a result, 1 compound out of 1134 candidate compounds hit, as can be seen in FIG. 7. This compound obtained by the screening was not among the 53 compounds obtained by the drug screening using the $MSD\delta_{t=500\,ms}$ ratio described above, and its effect on EGFR has not yet been reported. The RTK such as the EGFR transduces the signal from outside the cell into the cell not only through the phosphorylation of the receptor itself but also through the multimer formation. The fluorescence intensity used for the KL divergence analysis is one of the indicators that can quantitatively evaluate the amount and change (difference) of the EGFR multimer formation. Therefore, it was indicated that the compound/drug screening is feasible when using the KLD value obtained from the fluorescence intensity distribution, one of the quantitative indicators to characterize the single-molecule dynamics of the EGFR, and that a new compound/drug that could not be detected by existing methods can be explored.

[Experimental Example 6] Single-molecule imaging using inhibitor 2

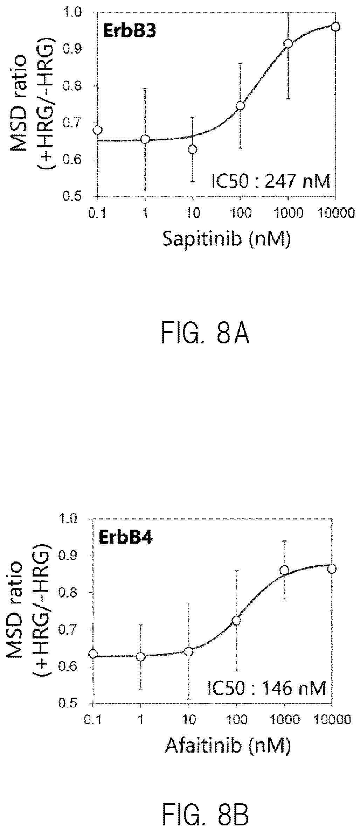

The CHO cell expressing the ErbB3-GFP was prepared, and the CHO cell expressing the ErbB3-GFP treated and untreated with HRG (30 to 100 nM) each were treated with Sapitinib, an inhibitor of the ErbB3, (concentrations: 0.1, 1, 10, 100, 1,000, and 10,000 nM), and then, single-molecule imaging was performed in the same way as Experimental Example 3; then, the $MSD\delta_{t=500\,ms}$ ($MSD_{HRG\text{-}treated}$ and $MSD_{HRG\text{-}untreated}$) for each drug concentration was calculated as for the treated and untreated groups, and further the $MSD\delta_{t=500\,ms}$ ratio was calculated using the following formula. The results are shown in FIG. 8A.

$$[\text{MSD}\delta_{t=500\text{ ms}}\ \text{ratio}]\ =\ \text{MSD}_{\text{HRG-treated}}\ /\ \text{MSD}_{\text{HRG-untreated}}$$

Single-molecule imaging was performed in the same way as above to calculate the $MSD\delta_{t=500\,ms}$ ratio, except that the CHO cell expressing the ErbB4-GFP and Afaitinib, which is an inhibitor of the ErbB4, were used. The results are shown in FIG. 8B.

FIGS. 8A and 8B show the relationship between the drug (Sapitinib or Afaitinib) concentration and the receptor diffusion dynamics ($MSD\delta_{t=500\,ms}$ ratio) when the HRG concentration is saturated (>30 nM); here, FIG. 8A is the result for Sapitinib and FIG. 8B is the result for Afaitinib. The $IC_{50}$ in FIGS. 8A and 8B is the 50% inhibitory concentration obtained from the $MSD\delta_{t=500\,ms}$ ratio. As can be seen in the graphs in FIGS. 8A and 8B, both Sapitinib and Afaitinib inhibited the reduction of the receptor mobility (ErbB3 or ErbB4) by HRG in a concentration-dependent manner. Therefore, it was confirmed that the effect of the phosphorylation inhibitor can be estimated from the change in the molecular dynamics by measuring the diffusion dynamics and calculating the ratio of the MSD values, even for the RTK receptor other than the EGFR.

**Claims**

1. A method for evaluating an activity of a receptor tyrosine kinase (RTK), the method comprising:

   a step (A1) for determining diffusion dynamics A1 of the RTK by contacting a target substance with a cell that expresses a labeled receptor tyrosine kinase (RTK) on a cell membrane;
   a step (B1) for determining diffusion dynamics B1 of the RTK by contacting the target substance and a ligand that activates the RTK with a cell that expresses the labeled receptor tyrosine kinase (RTK) on a cell membrane;
   a step (C1) for calculating a ratio of the diffusion dynamics A1 of the RTK obtained at the step (A1) and the diffusion dynamics B1 of the RTK obtained at the step (B1) (diffusion dynamics B1 / diffusion dynamics A1); and
   a step (D1) for evaluating an effect of the target substance on the RTK activity by comparing the ratio of the diffusion dynamics (diffusion dynamics B1 / diffusion dynamics A1) obtained at the step (C1) with a threshold, wherein
   at the step (D1), the ratio of the diffusion dynamics (diffusion dynamics B1 / diffusion dynamics A1) obtained at the step (C1) being equal to or greater than the threshold can indicate that the target substance has an effect on a molecular function of the RTK.

2. The method for evaluating the activity according to claim 1, wherein the diffusion dynamics is determined as a mean square displacement or a diffusion coefficient.

3. The method for evaluating the activity according to claim 1, further comprising:
   a step (E1) for determining diffusion dynamics E1 of the RTK without contacting the target substance and the ligand that activates the RTK with the cell that expresses the RTK on a cell membrane;

   a step (F1) for determining diffusion dynamics F1 of the RTK by contacting the ligand that activates the RTK, without contacting the target substance, with the cell that expresses the RTK on a cell membrane; and
   a step (G1) for calculating a ratio of the diffusion dynamics E1 of the RTK obtained at the step (E1) and the diffusion dynamics F1 of the RTK obtained at the step (F1) (diffusion dynamics F1 / diffusion dynamics E1), wherein
   the comparison of the ratio of the diffusion dynamics (diffusion dynamics B1 / diffusion dynamics A1) with the threshold at the step (D1) includes calculation of a difference between the ratios of the diffusion dynamics obtained at the step (C1) (diffusion dynamics B1 / diffusion dynamics A1) and the ratio of the diffusion dynamics obtained at the step (G1) (diffusion dynamics F1 / diffusion dynamics E1) ([diffusion dynamics B1 / diffusion dynamics A1] - [diffusion dynamics F1 / diffusion dynamics E1]), and comparison of the difference of the obtained ratios of the diffusion dynamics ([diffusion dynamics B1 / diffusion dynamics A1] - [diffusion dynamics F1 / diffusion dynamics E1]) with a threshold, and

the difference between the ratios of the diffusion dynamics ([diffusion dynamics B1 / diffusion dynamics A1] - [diffusion dynamics F1 / diffusion dynamics E1]) being equal to or greater than the threshold can be evaluated to indicate that the target substance has an effect on the molecular function of the RTK.

4. The method for evaluating the activity according to claim 1, wherein the label is a fluorescent label and the diffusion dynamics is measured using fluorescent single-molecule imaging.

5. A method for evaluating an activity of a receptor tyrosine kinase (RTK), the method comprising:

a step (A2) for determining diffusion dynamics A2 of the RTK without contacting a target substance with a cell that expresses a labeled receptor tyrosine kinase (RTK) on a cell membrane;
a step (B2) for determining diffusion dynamics B2 of the RTK by contacting the target substance with the cell that expresses the labeled receptor tyrosine kinase (RTK) on a cell membrane;
a step (C2) for calculating a ratio of the diffusion dynamics A2 of the RTK obtained at the step (A2) and the diffusion dynamics B2 of the RTK obtained at the step (B2) (diffusion dynamics B2 / diffusion dynamics A2), or a difference between the diffusion dynamics B2 and the diffusion dynamics A2 (diffusion dynamics B2 - diffusion dynamics A2);
a step (E2) for determining diffusion dynamics E2 of the RTK without contacting the target substance with the cell that expresses the labeled receptor tyrosine kinase (RTK) on a cell membrane;
a step (F2) for determining diffusion dynamics F2 of the RTK by contacting a solution not containing the target substance with the cell at the step (E2);
a step (G2) for calculating a ratio of the diffusion dynamics E2 of the RTK obtained at the step (E2) and the diffusion dynamics F2 of the RTK obtained at the step (F2) (diffusion dynamics F2 / diffusion dynamics E2), or a difference between the diffusion dynamics E2 and the diffusion dynamics F2 (diffusion dynamics F2 - diffusion dynamics E2); and
a step (D2) for evaluating the RTK activity of the target substance by comparing an absolute value of a difference between the ratios of the diffusion dynamics obtained at the step (C2) (diffusion dynamics B2 / diffusion dynamics A2) and the ratio of the diffusion dynamics obtained at the step (G2) (diffusion dynamics F2 / diffusion dynamics E2) ([diffusion dynamics B2 / diffusion dynamics A2] - [diffusion dynamics F2 / diffusion dynamics E2]), or an absolute value of a difference between the difference of the diffusion dynamics obtained at the step (C2) (diffusion dynamics B2 - diffusion dynamics A2) and the difference of the diffusion dynamics obtained at the step (G2) (diffusion dynamics F2 - diffusion dynamics E2) ([diffusion dynamics B2 - diffusion dynamics A2] - [diffusion dynamics F2 - diffusion dynamics E2]), with a threshold, wherein
at the step (D2), the absolute value of the difference between the ratios of the diffusion dynamics or the absolute value of the difference in the diffusion dynamics obtained at the step (G2) being equal to or greater than the threshold can indicate that the target substance has an effect on the RTK's molecular function not limited to a phosphorylation activity.

6. The method for evaluating the activity according to claim 5, wherein the diffusion dynamics is determined as a mean square displacement or a diffusion coefficient.

7. The method for evaluating the activity according to claim 5, wherein the label is a fluorescent label and the diffusion dynamics is measured using fluorescent single-molecule imaging.

8. A method for determining a quantified value of a molecular function and/or an activity of a receptor tyrosine kinase (RTK), and/or a 50% effective concentration ($EC_{50}$) of a target substance or a ligand on the molecular function and/or activity of the RTK, the method comprising:

a step (A3) for determining diffusion dynamics A3 of the RTK without contacting a target substance and a ligand that activates the RTK with a cell that expresses a labeled receptor tyrosine kinase (RTK) on a cell membrane;
a step (B3) for determining diffusion dynamics B3 of the RTK by contacting the target substance with the cell that expresses the labeled receptor tyrosine kinase (RTK) on a cell membrane or the ligand that activates the RTK;

a step (C3) for calculating a ratio of the diffusion dynamics A3 of the RTK obtained at the step (A3) and the diffusion dynamics B3 of the RTK obtained at the step (B3), (diffusion dynamics B3 / diffusion dynamics A3); and

a step (D3) for quantifying a value of an effect on the molecular function and/or activity of the RTK caused by the target substance or the ligand, and/or determining the 50% effective concentration ($EC_{50}$) of the target substance or the ligand on the molecular function and/or activity of the RTK, using the ratio of the diffusion dynamics (diffusion dynamics B3 / diffusion dynamics A3) obtained at the step (C3).

9. A method for evaluating an activity of a receptor tyrosine kinase (RTK), the method comprising:

a step (A4) for contacting a target substance with a cell that expresses a labeled receptor tyrosine kinase (RTK) on a cell membrane;

a step (B4) for contacting the target substance and a ligand that activates the RTK with the cell that expresses the labeled receptor tyrosine kinase (RTK) on a cell membrane;

a step (C4) for quantifying distributions of the degree of RTK multimerization at the step (A4) and at the step (B4); and

a step (D4) for evaluating the RTK activity of the target substance by comparing the value obtained at the step (C4) with a threshold, wherein

a difference between the value obtained at the step (C4) and the threshold being equal to or greater than a predetermined value at the step (D4) can indicate that the target substance has an effect on a molecular function of the RTK.

10. The method for evaluating the activity according to claim 9, wherein the distribution at the step (C4) is determined as a distribution of luminance of the label or a distribution of a diffusion coefficient of the label.

11. The method for evaluating the activity according to claim 9, wherein the quantification at the step (C4) is performed by KL divergence.

12. The method for evaluating the activity according to claim 9, wherein the label is a fluorescent label and the distribution is measured using fluorescent single-molecule imaging.

13. A method for evaluating an activity of a receptor tyrosine kinase (RTK), the method comprising:

a step (A5) for contacting a target substance with a cell that expresses a labeled receptor tyrosine kinase (RTK) on a cell membrane;

a step (B5) for quantifying a distribution of the degree of RTK multimerization before and after the contact of the target substance; and

a step (C5) for evaluating the target substance on a molecular function and/or activity of the RTK by comparing the value obtained at the step (B5) with a threshold, wherein

a difference between the value obtained at the step (B5) and the threshold being equal to or greater than a predetermined value at the step (C5) can indicate that the target substance has an effect on the molecular function and/or activity of the RTK.

14. The method for evaluating the activity according to claim 13, wherein the distribution at the step (B5) is determined as a distribution of luminance of the label or a distribution of a diffusion coefficient of the label.

15. The method for evaluating the activity according to claim 13, wherein the quantification at the step (B5) is performed by KL divergence.

16. The method for evaluating the activity according to claim 13, wherein the label is a fluorescent label and the distribution is measured using fluorescent single-molecule imaging.

17. The method for evaluating the activity according to any of claims 1 to 7 and 9 to 16, wherein the RTK is an epidermal growth factor receptor (EGFR).

18. A method for screening a candidate inhibitor or activator targeting the RTK, the method comprising evaluating a target substance by the method for evaluating the activity according to any of claims 1 to 7 and 9 to 16, and/or determining a quantified value of a molecular function and/or activity of the RTK and/or a 50% effective concentration ($EC_{50}$) of the target substance or the ligand on the molecular function and/or activity of the RTK according to claim 8.

FIG. 1

FIG. 2

FIG. 3

FIG. 4A

FIG. 4B

FIG. 5

FIG. 6

FIG. 7

FIG. 8A

FIG. 8B

**EP 4 674 975 A1**

## INTERNATIONAL SEARCH REPORT

International application No.

**PCT/JP2024/007599**

### A. CLASSIFICATION OF SUBJECT MATTER

*C12Q 1/48*(2006.01)i; *C12Q 1/02*(2006.01)i; *G01N 33/15*(2006.01)i; *G01N 33/50*(2006.01)i; *G01N 33/573*(2006.01)i
FI: C12Q1/48 Z; G01N33/15 Z; G01N33/573 A; G01N33/50 Z; C12Q1/02

According to International Patent Classification (IPC) or to both national classification and IPC

### B. FIELDS SEARCHED

Minimum documentation searched (classification system followed by classification symbols)

C12Q1/48; C12Q1/02; G01N33/15; G01N33/50; G01N33/573

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | YASUI, M. et al. Automated single-molecule imaging in living cells. NATURE COMMUNICATIONS. 2018, vol. 9, article no. 3061, pp. 1-11, DOI: 10.1038/s41467-018-05524-7<br>p. 1, summary, p. 4, right column, line 5 to p. 5, lower right column, line 1 from the bottom, p. 6, left column, line 1 to lower right column, line 6, fig. 1, 5 | 1-4, 8-10, 12-14, 16-18 |
| Y | p. 1, summary, p. 4, right column, line 5 to p. 5, lower right column, line 1 from the bottom, p. 6, left column, line 1 to lower right column, line 6, fig. 1, 5 | 11, 15, 17, 18 |
| A |  | 5-7 |

☑ Further documents are listed in the continuation of Box C.     ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **26 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/007599**

## C. DOCUMENTS CONSIDERED TO BE RELEVANT

| Category* | Citation of document, with indication, where appropriate, of the relevant passages | Relevant to claim No. |
|---|---|---|
| X | 上田昌宏, 1分子自動イメージング法の開発と細胞への適用、細胞内1分子スクリーニングの実現に向けて, 日本の科学者, 2019, vol. 54, no. 5, pp. 11-17, (UEDA, Masahiro. Journal of Japanese scientists.), non-official translation (Development of automatic single-molecule imaging method and its application to cells. Towards the realization of in-cell single-molecule screening.)<br>　　p.13, lower right column, line 6 from the bottom to p. 14, left column, line 5, p. 16, left column, line 12 from the bottom to right column, line 16, fig. 2, 3 | 1-4, 8, 17, 18 |
| Y | 　　p.13, lower right column, line 6 from the bottom to p. 14, left column, line 5, p. 16, left column, line 12 from the bottom to right column, line 16, fig. 2, 3 | 9-18 |
| A | | 5-7 |
| Y | YANAGAWA, M. et al. Single-molecule diffusion-based estimation of ligand effects on G protein-coupled receptors. Science Signaling. 2018, vol. 11, no. 548, eaao1917, pp. 1-16, DOI: 10.1126/scisignal.aao1917<br>　　p. 13, left column, line 15 to p. 14, right column, line 15, fig. 2 | 9-18 |
| A | JP 2018-179946 A (RIKEN) 15 November 2018 (2018-11-15)<br>　　claims, abstract | 1-18 |

Form PCT/ISA/210 (second sheet) (July 2022)

34

## INTERNATIONAL SEARCH REPORT
### Information on patent family members

International application No.

**PCT/JP2024/007599**

| Patent document cited in search report | | | Publication date (day/month/year) | Patent family member(s) | | | Publication date (day/month/year) |
|---|---|---|---|---|---|---|---|
| JP | 2018-179946 | A | 15 November 2018 | US | 2018/0306779 | A1 | |
| | | | | claims, abstract | | | |

Form PCT/ISA/210 (patent family annex) (July 2022)

REFERENCES CITED IN THE DESCRIPTION

**Patent documents cited in the description**

- JP 2018179946 A
- WO 2019159627 A

- JP 2021029165 A

**Non-patent literature cited in the description**

- **MASATO YASUI et al.** *nature commun*, 2018
- **M. YANAGAWA et al.** *Science Signaling*, 18 September 2018, vol. 11, eaao1917

- **M. YASUI et al.** *nature commun*, 2018, vol. 9, 3061
- **M. HIROSHIMA et al.** *Microscopy*, 2020, vol. 69 (2), 69-78